(19) Europäisches Patentamt
European Patent Office
Office européen des brevets

(11) Numéro de publication : **0 044 260**
**B1**

(12) # FASCICULE DE BREVET EUROPÉEN

(45) Date de publication du fascicule du brevet : **11.04.84**

(21) Numéro de dépôt : **81420099.4**

(22) Date de dépôt : **06.07.81**

(51) Int. Cl.$^3$ : **C 07 C 47/565**, C 07 C 47/575, C 07 C 39/08, C 07 C 39/10, C 07 C 39/11, C 07 C 43/23, C 07 C 45/67, C 07 C 45/38, C 07 C 37/56, C 07 C 37/20, C 07 C 41/26

(54) **Procédé de préparation de polyphénols comportant éventuellement un groupement aldéhyde.**

(30) Priorité : **11.07.80 FR 8015753**

(43) Date de publication de la demande : **20.01.82 Bulletin 82/03**

(45) Mention de la délivrance du brevet : **11.04.84 Bulletin 84/15**

(84) Etats contractants désignés : **AT BE CH DE FR GB IT LI LU NL SE**

(56) Documents cités :
**FR-A- 1 189 191**
**FR-A- 2 075 617**
**FR-A- 2 155 074**
**FR-A- 2 305 420**
**FR-A- 2 350 323**
**US-A- 1 891 149**
**US-A- 1 965 458**
**US-A- 3 585 243**
**US-A- 3 673 257**
**Le dossier contient des informations techniques présentées postérieurement au dépôt de la demande et ne figurant pas dans le présent fascicule.**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Formanek, Karel**
**3, rue de la Sarrazinière**
**F-69360 Serezin du Rhône (FR)**
Inventeur : **Michelet, Daniel**
**24, chemin de Montribloud**
**F-69160 Tassin la demi Lune (FR)**
Inventeur : **Petre, Dominique**
**31, rue Boileau**
**F-69006 Lyon (FR)**

(74) Mandataire : **Rioufrays, Roger et al**
**RHONE-POULENC RECHERCHES Centre de Recherches de Saint-Fons Service Brevets B.P. 62**
**F-69190 Saint-Fons (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 044 260 B1

# Procédé de préparation de polyphénols comportant éventuellement un groupement aldéhyde

La présente invention a pour objet un procédé de préparation de polyphénols comportant au moins deux groupes hydroxyle et éventuellement un groupe aldéhyde par oxydation d'hydroxybenzaldéhydes par l'eau oxygénée.

Les polyphénols substitués ou non par des groupes fonctionnels tels que les groupes aldéhydes et/ou alkoxy sont des produits industriels très recherchés. Ainsi l'hydroquinone est utilisée notamment dans l'industrie photographique. La pyrocatéchine est de son côté une matière première particulièrement importante pour la préparation de composés organiques tels que le gaïacol et ses dérivés ou la préparation de résines par condensation avec le formol. Le pyrogallol est également employé comme révélateur photographique et comme intermédiaire en synthèse organique. Il en est de même pour l'aldéhyde protocatéchique (dihydroxy-3,4 benzaldéhyde), l'hydroxy-para-vanilline (dihydroxy-3,4 méthoxy-5 benzaldéhyde) et l'hydroxy-ortho-vanilline (dihydroxy-2,5 méthoxy-3 benzaldéhyde).

On a proposé de nombreux procédés de préparation des polyphénols. Parmi ces procédés on peut en signaler deux groupes qui ont pour point commun l'oxydation d'un composé phénolique par l'eau oxygénée et les peroxydes qui en dérivent et notamment les peracides minéraux ou organiques.

Ces deux groupes de procédés se distinguent par la nature des produits de départ. Ainsi on sait préparer des polyphénols par hydroxylation directe du noyau aromatique par l'eau oxygénée ou les peracides organiques tels que les acides performique et peracétique. De tels procédés d'hydroxylation qui conviennent tout particulièrement bien à la préparation de l'hydroquinone et de la pyrocatéchine, ont été décrits notamment dans les brevets français n° 69.45467 publié sous le n° 2.071.464, n° 75.40382 publié sous le n° 2.336.364 et n° 1.479.354. Bien que ces procédés se révèlent d'un grand intérêt il peut être avantageux dans certains cas de faire appel à un second groupe de procédés dont la caractéristique réside dans la substitution d'un ou plusieurs groupes aldéhyde d'un benzaldéhyde par un ou plusieurs groupes hydroxyles.

L'oxydation des aldéhydes aromatiques en les phénols correspondants au moyen d'eau oxygénée ou d'acides percarboxyliques, connue généralement sous le nom de réaction de BAYER et VILLIGER est un moyen commode d'obtention d'un phénol lorsqu'on dispose d'une source d'aldéhydes aromatiques. Cf. C. H. HASSAL Organic Reactions Volume 9 p. 73 à 106 (1957). La réaction de BAYER et VILLIGER couvre en réalité deux types de réaction. Dans le premier figure l'oxydation des aldéhydes aromatiques par les acides percarboxyliques formés « in situ » ou extemporanément par réaction de l'eau oxygénée avec un acide carboxylique tels que les acides formique, acétique ou benzoïque. Dans tous les cas cette oxydation peut être conduite en présence d'un acide fort comme catalyseur (acide toluène-sulfonique par exemple). Cette réaction ne conduit pas aux phénols au départ de tous les aldéhydes aromatiques. On a constaté en effet que le benzaldéhyde et ses homologues comportant des substituants électroattracteurs (atomes d'halogènes, groupe nitro par exemple) sont oxydés par les peracides en les acides benzoïques correspondants alors que les aldéhydes aromatiques comportant des substituants électrodonneurs (groupes hydroxyle, alcoxyle, alkyle) tels que le salicylaldéhyde, le p-hydroxybenzaldéhyde, l'ortho et le p-méthoxybenzaldéhyde conduisent aux phénols correspondants (éventuellement sous forme de formiates, suivant les conditions de la réaction), cf J. BOESEKEN et al. Rec. Trav. Chim. Pays-Bas *55* 815 (1936) ; J. BOESEKEN et al. Ibid. *74* 845 (1941) ; Y. OGATA et al. J. Org. Chem. *26* 4803 (1961). Ce procédé d'oxydation en phénols des aldéhydes aromatiques à substituants électrodonneurs présente l'inconvénient de nécessiter l'intervention d'un acide carboxylique qui joue en fait le rôle d'un vecteur d'oxygène actif dans la réaction. La présence de l'acide carboxylique dans le processus implique la mise en œuvre de volumes réactionnels importants qui limitent, au plan industriel, la productivité de la réaction. Par ailleurs, le fait d'opérer en milieu anhydre pour favoriser la formation des peracides crée des risques d'explosion. Il est donc souhaitable de s'affranchir de l'intervention de tout acide carboxylique et par conséquent de mettre en œuvre directement l'eau oxygénée comme source d'oxygène actif. Un tel but est atteint en conduisant la réaction en milieu alcalin : il s'agit alors du second groupe de procédés assimilés à la réaction de BAYER et VILLIGER et généralement désignés sous le nom de réaction de DAKIN. Selon ce dernier cf. Am. chem. J. *42* 474 (1909) les aldéhydes aromatiques comportant un ou plusieurs groupes hydroxyle en ortho ou en para du groupe carbonyle sont oxydés avec de bons rendements en les polyphénols correspondants par l'eau oxygénée en milieu alcalin. Dans ces conditions les groupes aldéhyde en position méta par rapport à l'hydroxyle phénolique ne sont pas oxydés. Ce procédé a été appliqué à de nombreux aldéhydes aromatiques comportant au moins un groupe hydroxyle en ortho ou para tels que notamment le salicylaldéhyde, le p-hydroxybenzaldéhyde, le dihydroxy-2,4 benzaldéhyde, l'hydroxy-2 méthoxy-3 benzaldéhyde, l'hydroxy-2 méthoxy-5 benzaldéhyde, l'hydroxy-3 méthoxy-5 benzaldéhyde. A la différence de la réaction de BAYER et VILLIGER proprement dite la réaction de DAKIN s'applique à des hydroxybenzaldéhydes substitués par des atomes d'halogènes tels que le dibromo-3,5 hydroxy-4 benzaldéhyde, le dichloro-3,5 hydroxy-4 benzaldéhyde ; le bromo-5 hydroxy-2 benzaldéhyde ; le bromo-3 hydroxy-4 méthoxy-5 benzaldéhyde et à certains nitrohydroxy benzaldéhydes comme le nitro-3 hydroxy-2 benzaldéhyde ; le nitro-5 hydroxy-2 benzaldéhyde ; le nitro-2 hydroxy-4 méthoxy-3 benzaldéhyde : cf. DAKIN loc. cit. ; DAKIN Org. Synth. Coll. vol. 1. p. 149 (1941) ; SURREY Org. Synth. *26* p. 90 (1945) ; J. KVALNES J. Am. Chem. Soc. *56* 2 487 (1934).

La réaction de DAKIN est conduite au sein d'une solution aqueuse d'une base alcaline telle que la soude, le pH du milieu étant fortement alcalin, généralement supérieur à 8. Dans ces conditions, on ne peut éviter une oxydation indésirable des produits de la réaction en quinones. De plus on a constaté que dans le cas d'hydroxybenzaldéhydes comportant à la fois un groupe aldéhyde en position para et au moins un groupe aldéhyde en position ortho par rapport à l'hydroxyle phénolique les rendements en produits résultant de la seule oxydation des groupes aldéhydes en ortho sont faibles en milieu alcalin même lorsque la quantité d'eau oxygénée est insuffisante pour oxyder la totalité des groupes aldéhydes présents. Il n'est donc pas possible dans les conditions habituelles de la réaction de DAKIN de préparer avec des rendements présentant un intérêt industriel des polyhydroxybenzaldéhydes par oxydation des seuls groupes aldéhydes en ortho de l'hydroxyle phénolique. Ainsi en est-il de l'oxydation de l'hydroxy-2 méthoxy-3 isophtalaldéhyde (diformylgaïacol) en ortho hydroxy p-vanilline. De la même façon il peut se révéler intéressant au plan industriel de pouvoir oxyder avec de bons rendements un mélange d'un orthohydroxy- et d'un parahydroxybenzaldéhyde tel que les mélanges de salicylaldéhyde et de p-hydroxybenzaldéhyde ou de vanillline et d'hydroxy-2 méthoxy-3 benzaldéhyde.

La présente invention concerne précisément un procédé d'oxydation des hydroxybenzaldéhydes en polyphénols par l'eau oxygénée, selon la réaction de DAKIN qui ne présente pas les inconvénients des procédés de l'art antérieur.

Plus spécifiquement la présente invention a pour objet un procédé de préparation de polyphénols comportant éventuellement un groupe aldéhyde par oxydation d'hydroxybenzaldéhydes comportant au moins un groupe aldéhyde en ortho et/ou para par rapport au groupe hydroxyle et répondant à la formule générale :

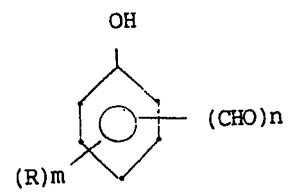

(I)

dans laquelle :

— n est un nombre entier de 1 à 3,

— R représente un radical alkyle, alkoxy, hydroxyalkyle, cycloalkyle, aryle, alkoxyalkyle, hydroxyle, un groupe nitro, un atome d'halogène,

— m est un nombre entier de 0 à 3, la somme m + n étant au plus égale à 4,

à l'exclusion des monohydroxybenzaldéhydes de formule (I) dans laquelle n est égal à 1, par l'eau oxygénée en milieu aqueux en présence d'une base alcaline ou alcalino-terreuse caractérisé en ce que le pH du milieu réactionnel est inférieur ou égal à 7 pendant la durée de la réaction.

On a constaté en effet de manière inattendue que le maintien du pH à une valeur inférieure ou égale à 7 permet d'éviter ou au moins de limiter l'oxydation par l'eau oxygénée des polyphénols formés dans la réaction en produits quinoniques pour tous les hydroxybenzaldéhydes mis en œuvre. Il permet également de limiter la dégradation de l'eau oxygénée en oxygène moléculaire et par conséquent de limiter la perte d'oxygène actif. Dans le cas d'hydroxybenzaldéhydes comportant à la fois au moins un groupe aldéhyde en ortho et un groupe aldéhyde en para par rapport à l'hydroxyle phénolique, le procédé selon l'invention se prête à la préparation avec d'excellents rendements des polyphénols ayant au moins un groupe hydroxyle en ortho et un groupe aldéhyde en para de l'hydroxyle initial.

Le choix du pH optimal de la phase aqueuse d'oxydation dépend de la nature de l'hydroxybenzaldéhyde mis en œuvre. Il est en effet préférable pour assurer un déroulement normal de la réaction de DAKIN que l'hydroxybenzaldéhyde peu soluble dans l'eau soit présent dans la phase aqueuse, sous forme de son sel alcalin ou alcalino-terreux ou, plus exactement que de l'hydroxybenzaldéhyde sous forme de son sel soit constamment disponible en phase aqueuse sans que pour autant le pH de cette dernière dépasse la limite de 7. En définitive ce pH optimal dépend du pKa dans l'eau de l'hydroxybenzaldéhyde considéré. Il va sans dire que la réaction conduit encore aux résultats recherchés lorsqu'on s'écarte quelque peu de part et d'autre de la valeur du pH correspondant au pKa de l'hydroxybenzaldéhyde considéré, à condition bien sûr de ne pas dépasser la valeur limite de 7. Ainsi le pH peut être inférieur à la valeur optimale correspondant au pKa de l'hydroxybenzaldéhyde. On constate cependant que plus le pH est acide plus la réaction est lente. Il n'est donc pas recommandé de conduire la réaction à pH inférieur à 2 bien que l'on ne sorte pas du domaine de la présente invention en opérant à des pH inférieurs à cette valeur.

En règle générale on choisit pour le pH du milieu d'oxydation une valeur comprise entre 2 et 7 et de préférence entre 3 et 6 pendant la durée de la réaction.

Dans la pratique le pH de la phase aqueuse contenant l'hydroxybenzaldéhyde est porté à la valeur désirée puis l'eau oxygénée est ajoutée. Pour éviter un abaissement progressif du pH dû à la formation d'acide formique il est préférable d'ajouter au fur et à mesure du déroulement de la réaction une quantité d'une base alcaline ou alcalino-terreuse suffisante pour éviter une acidification trop grande du milieu réactionnel. En général la quantité de base alcaline est calculée pour maintenir le pH au voisinage de la valeur optimale choisie.

Plus spécifiquement dans la formule (I) R représente un radical alkyle ayant de 1 à 20 atomes de carbone tel que les radicaux méthyle, éthyle, n-propyle, isopropyle, n-butyle, iso-butyle, t-butyle, pentyles, décyles, pentadécyles ; un radical alkoxy ayant de 1 à 20 atomes de carbone tel que les radicaux méthoxy, éthoxy, n-propyloxy, isopropyloxy, n-butyloxy, t-butyloxy, pentyloxy, dodécyloxy, hexadécyloxy ; un radical cycloalkyle comme les radicaux cyclopentyle et cyclohexyle ; un radical aryle comme les radicaux phényle, toluyle, xylyle ; un radical alkoxyalkyle ayant de 1 à 20 atomes de carbone au total tel que les groupes β-méthoxyéthyle, β-éthoxyéthyle, méthoxy-3 n-propyle ; un atome d'halogène tel que le chlore et le brome.

De préférence dans la formule (I) R représente un radical alkyle inférieur comportant de 1 à 4 atomes de carbone, un radical alkoxy inférieur comportant de 1 à 4 atomes de carbone, un groupe hydroxyle ; m est 0 ou 1 ; plus préférentiellement encore n est égal à 2 ou 3.

Le procédé selon la présente invention permet d'obtenir à partir des hydroxybenzaldéhydes de formule (I) et avec les avantages rapportés plus haut, des polyphénols comportant au moins un groupe hydroxyle de plus que le composé soumis à l'oxydation et, le cas échéant, un groupe aldéhyde résiduel en position para par rapport à l'hydroxyle phénolique initial.

Plus spécifiquement la présente invention a pour objet un procédé de préparation de polyphénols comportant éventuellement un groupe aldéhyde de formule générale :

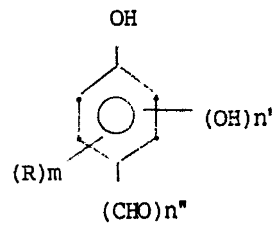

(II)

dans laquelle :

— R et m ont la signification déjà donnée,

— n' est un nombre entier de 1 à 2

— n'' est 0 ou 1, la somme n' + n'' étant égale à n

les groupes hydroxyles engendrés au cours de l'oxydation étant en position ortho ou le cas échéant en position para par rapport à l'hydroxyle phénolique initial.

Parmi les hydroxybenzaldéhydes de formule (I) auxquels on peut appliquer le procédé selon l'invention, on distingue les familles suivantes :

a) Les hydroxybenzaldéhydes comportant un ou deux groupes aldéhydes juxtanucléaires en position ortho par rapport à l'hydroxyle phénolique répondant à la formule générale :

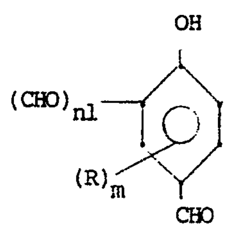

(III)

dans laquelle R et m ont la signification déjà donnée et $n_1$ est 0 ou 1. Ils conduisent à l'obtention de polyphénols de formule générale :

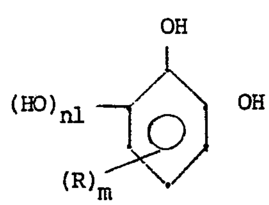

(IV)

dans laquelle R et m ont la signification déjà donnée.

Par la suite les différents symboles R, m, $n_1$, n' et n'' utilisés pour les formules (I) à (IV) conserveront les définitions données ci-avant sauf indication contraire.

b) Les hydroxybenzaldéhydes comportant un groupe aldéhyde en position para par rapport à l'hydroxyle phénolique, de formule générale :

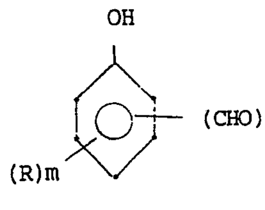

(V)

Ces hydroxybenzaldéhydes conduisent aux paradiphénols correspondants.

Le procédé selon la présente invention s'applique tout particulièrement bien à l'oxydation de mélanges d'aldéhydes de formules (III) et (V) tels que ceux obtenus par hydroxyméthylation du phénol ou des phénols substitués au moyen du formaldéhyde ou de ses dérivés puis oxydation des mélanges des hydroxyméthylphénols ainsi obtenus. On peut citer en particulier les mélanges aldéhyde salicylique/p-hydroxybenzaldéhyde.

Il convient également particulièrement bien à l'oxydation des hydroxybenzaldéhydes de formules (III) et (V) dans lesquelles R représente un groupe hydroxyle ou un radical alkoxy inférieur et m est égal à au moins 1 et à leurs mélanges, tels que l'aldéhyde protocatéchique, le dihydroxy-2,3 benzaldéhyde et les mélanges aldéhyde protocatéchique/dihydroxy-2,3 benzaldéhyde. Lors de l'oxydation de tels mélanges le recours à un pH inférieur à 7 permet d'améliorer les rendements en polyphénols dérivés de ces hydroxybenzaldéhydes dans de notables proportions.

c) Les hydroxybenzaldéhydes comportant à la fois un ou deux groupes aldéhydes juxtanucléaires en position ortho par rapport à l'hydroxyle phénolique et un groupe aldéhyde en position para. Ces hydroxybenzaldéhydes qui répondent plus spécifiquement à la formule générale :

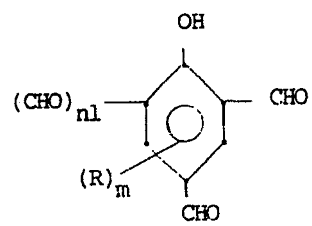

(VI)

conduisent à l'obtention de polyhydroxybenzaldéhydes de formule générale :

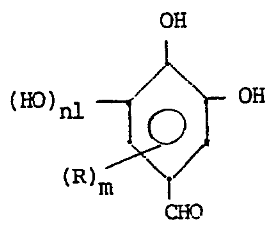

(VII)

Le procédé selon la présente invention convient tout particulièrement bien à la préparation des polyhydroxybenzaldéhydes de formule (VII) à partir des hydroxybenzaldéhydes de formule (VI) car à pH inférieur à 7 les groupes aldéhydes en ortho de l'hydroxyle phénolique sont oxydés plus rapidement que le groupe aldéhyde en position para. Il devient donc possible d'oxyder sélectivement les premiers en évitant ou au moins en limitant fortement l'oxydation du second.

Dans ce cas le procédé selon l'invention permet d'accéder à des dihydroxy-3,4 ou trihydroxy-3,4,5 benzaldéhydes, intermédiaires recherchés en synthèse organique.

Parmi les hydroxybenzaldéhydes auxquels on peut appliquer le procédé selon l'invention on peut citer à titre non limitatif :

— le dihydroxy-2,3 benzaldéhyde,
— le dihydroxy-2,6 benzaldéhyde,
— l'aldéhyde protocatéchique,
— le diformyl-2,4 phénol,
— le diformyl-2,6 phénol,
— le dihydroxy-1,2 diformyl-3,5 benzène,
— le dihydroxy-1,2 diformyl-4,6 benzène,
— l'hydroxy-1 méthoxy-2 diformyl-4,6 benzène (diformyl-4,6 gaïacol),
— l'hydroxy-1 éthoxy-2 diformyl-4,6 benzène.

Parmi les polyhydroxybenzaldéhydes qui peuvent être préparés par le procédé selon la présente invention on peut citer à titre non limitatif :

- l'aldéhyde protocatéchique,
- l'orthohydroxy para-vanilline,
- le dihydroxy-3,4 éthoxy-5 benzaldéhyde,
- le trihydroxy-3,4,5 benzaldéhyde,
- le dihydroxy-3,4 méthoxy-5 benzaldéhyde,
- le pyrogallol.

Le procédé selon l'invention convient tout particulièrement bien à l'oxydation du diformyl-2,4 phénol en aldéhyde protocatéchique (dihydroxy-3,4 benzaldéhyde), du diformylgaïacol en dihydroxy-3,4 méthoxy-5 benzaldéhyde, du dihydroxy-2,6 benzaldéhyde en pyrogallol, du diformyl-4,6 éthoxy-2 phénol en dihydroxy-3,4 éthoxy-5 benzaldéhyde, du dihydroxy-1,2 diformyl-4,6 benzène en trihydroxy-3,4,5 benzaldéhyde ; du triformyl-2,4,6 phénol en trihydroxy-3,4,5 benzaldéhyde.

La température à laquelle on conduit la réaction peut varier dans de larges limites. En général des températures comprises entre 0 °C et la température d'ébullition du milieu réactionnel conviennent bien. Dans la plupart des cas on fait appel à des températures allant de 10 à 100 °C et de préférence de 30 à 80 °C.

La concentration de l'hydroxybenzaldéhyde en suspension dans le milieu réactionnel n'est pas critique et peut varier dans de larges limites. Elle peut être comprise entre 0,1 et 10 moles par litre et de préférence entre 0,2 et 2 moles par litre. On peut cependant opérer en dehors de ces limites sans sortir du cadre de la présente invention.

La quantité d'eau oxygénée exprimée en moles de $H_2O_2$ par groupe aldéhyde à oxyder peut varier dans de larges limites. On peut donc opérer avec un défaut ou un excès d'eau oxygénée par rapport à la quantité théorique. En général on utilise des quantités d'eau oxygénée vosines de la stœchiométrie de la réaction c'est-à-dire de 1 mole de $H_2O_2$ par groupe aldéhyde à oxyder. Dans le cas d'hydroxybenzaldéhydes ne comportant qu'un groupe aldéhyde qu'il soit en position ortho ou para ou comportant deux groupes aldéhydes en position ortho on peut faire appel à un gros excès d'eau oxygénée mais sans que cela apporte d'avantage particulier. Lorsqu'on oxyde des hydroxybenzaldéhydes comportant à la fois un groupe aldéhyde en para et un ou deux groupes en ortho, il est préférable de limiter l'excès d'eau oxygénée par rapport à la quantité stœchiométrique pour l'oxydation des groupes aldéhydes en ortho. En pratique il est avantageux dans ce cas de recourir à une quantité d'eau oxygénée d'au plus 1,6 mole par groupe aldéhyde en position ortho. On peut également opérer avec un défaut d'eau oxygénée mais alors on limite le taux de transformation de l'hydroxybenzaldéhyde de départ. En définitive une quantité d'eau oxygénée comprise entre 0,5 et 1,8 mole de $H_2O_2$ par groupe aldéhyde à oxyder convient bien ; de préférence elle est comprise entre 1 et 1,5.

La concentration de la solution aqueuse d'eau oxygénée n'est pas critique. Pour des raisons pratiques on a recours aux solutions courantes dont la concentration est comprise entre 20 et 75 % en poids.

Parmi les bases alcalines ou alcalino-terreuses auxquelles on peut faire appel pour mettre en œuvre le présent procédé, on peut citer des hydroxydes tels que la soude, la potasse, l'hydroxyde de lithium et la baryte ; des alcanolates alcalins tels que le méthylate, l'éthylate, l'isopropylate et le t-butylate de sodium ou de potassium, des carbonates ou bicarbonates de sodium ou de potassium et de façon générale les sels des bases alcalines ou alcalino-terreuses et d'acides faibles.

La quantité de base alcaline est calculée pour que le pH du milieu se situe au-dessous de la valeur limite définie précédemment. Elle est déterminée par le pKa dans l'eau de l'hydroxybenzaldéhyde soumis à l'oxydation.

Le procédé selon la présente invention est de préférence conduit sous atmosphère d'un gaz inerte tel que l'azote ou l'argon.

Les polyphénols obtenus par oxydation des hydroxybenzaldéhydes par le procédé selon l'invention peuvent être séparés du milieu réactionnel par les procédés usuels. On peut par exemple traiter le milieu d'oxydation par une base alcaline pour saponifier complètement le formiate de phényle formé de façon à libérer le phénol puis séparer ce dernier par les procédés usuels par exemple par extraction.

Il peut être avantageux lorsque le produit obtenu est destiné à la préparation de composés dérivés de ne pas procéder à la séparation du polyphénol obtenu et d'utiliser la masse réactionnelle directement pour l'opération suivante. Ainsi lorsqu'on désire préparer des éthers dérivés des polyphénols obtenus, par alkylation au moyen d'un agent alkylant tel que les halogénures et sulfates d'alkyle en milieu alcalin on peut ajouter au milieu d'oxydation brut une quantité suffisante d'une base alcaline telle que celles citées auparavant pour porter le pH à une valeur supérieure à 8 puis conduire la réaction d'alkylation dans les conditions usuelles. Un tel procédé peut être utilisé en particulier pour l'éthérification des polyhydroxybenzaldéhydes de formule (VII). C'est ainsi qu'en enchaînant l'étape d'oxydation et d'éthérification on peut préparer le triméthoxybenzaldéhyde à partir du diformylgaïacol.

Les hydroxybenzaldéhydes de formule (I) auxquels on peut appliquer le procédé selon l'invention

sont des produits généralement connus à l'exception du triformyl-2,4,6 phénol qui n'a pas été décrit dans la littérature, qui peuvent être préparés par diverses méthodes de synthèse organique.

Ainsi les hydroxybenzaldéhydes peuvent-ils être préparés par oxydation de méthylolphénols par l'oxygène moléculaire ou un gaz qui en contient en phase aqueuse alcaline en présence d'un catalyseur à base d'un métal noble tel que le platine et le palladium, contenant éventuellement à titre d'activateur des métaux tels que le cadmium, le cérium, le bismuth, le plomb, l'argent, le tellure ou l'étain. De tels procédés ont été décrits dans le brevet américain 3.673.257, le brevet français n° 75.09932 publié sous le n° 2 305 420 et la demande de brevet français n° 77.13941 publiée sous le n° 2 350 323.

De leur côté les méthylolphénols sont des produits connus pour la plupart et qui peuvent être préparés par hydroxyméthylation de phénols substitués ou non au moyen du formaldéhyde ou de composés générateurs de formaldéhyde comme le paraformaldéhyde, dans les conditions les plus diverses : cf notamment H. G. PEER Rec. Trav. Chim. Pays-Bas *79* 825-835 (1960) ; brevet anglais 774.696 ; brevet anglais 751.845 ; demande de brevet européen n° 165 ; J. H. FREEMAN J. Am. Chem. Soc. *74* 6257-6260 (1952) et *76* 2080-2087 (1954) ; H. G. PEER Rec. Trav. Chim. Pays-Bas *78* 851-863 (1959) ; H. EULER et al. Arkiv für Chem. *13* 1-7 (1939) ; P. CLAUS et al. Monath. Chem. *103* 1178-1193 (1972).

Un procédé d'hydroxyméthylation des phénols qui convient tout spécialement bien à la synthèse des méthylphénols utilisables pour la préparation des hydroxybenzaldéhydes réside dans la condensation de formaldéhyde ou des composés générateurs de formaldéhyde avec un phénol en phase aqueuse en présence d'une base alcaline ou alcalino-terreuse.

Il apparaît particulièrement avantageux du point de vue industriel pour la mise en œuvre du procédé selon la présente invention de faire appel à des hydroxybenzaldéhydes obtenus par un procédé en deux étapes comprenant a) l'hydroxyméthylation d'un phénol en milieu aqueux en présence d'une base alcaline ou alcalino-terreuse par le formaldéhyde ou un composé générateur de formaldéhyde, en un mono ou polyméthylolphénol et b) l'oxydation, sans séparation intermédiaire, des méthylolphénols par l'oxygène moléculaire ou un gaz qui en contient en phase aqueuse alcaline en présence d'un catalyseur au palladium ou au platine contenant éventuellement à titre d'activateur un métal tel que ceux cités précédemment.

Le procédé de l'invention s'applique tout spécialement bien à des hydroxybenzaldéhydes de formule (I) obtenus par un procédé en deux étapes comprenant :

1. l'hydroxyméthylation de phénols de formule générale :

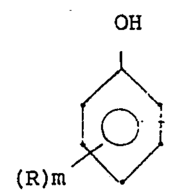

(VIII)

non substitués sur l'une au moins des positions ortho par rapport à l'hydroxyle phénolique et éventuellement en position para, en méthylolphénols de formule générale :

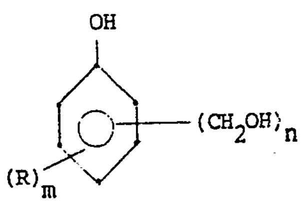

(IX)

comportant au moins un groupe méthylol en position ortho et/ou para par rapport au groupe hydroxyle, au moyen du formaldéhyde ou d'un générateur de formaldéhyde en phase aqueuse en présence d'une base alcaline ou alcalino-terreuse ;

2. l'oxydation en phase aqueuse alcaline des méthylolphénols de formule (IX) résultants de la première étape, par l'oxygène moléculaire ou un gaz qui en contient en présence d'un catalyseur au palladium ou au platine contenant éventuellement un métal tel que ceux utilisés dans l'art antérieur à titre d'activateur, sans séparation intermédiaire des méthylolphénols.

Plus spécifiquement encore le procédé selon la présente invention convient bien à la préparation des polyphénols à groupe aldéhyde de formule (VII) à partir d'hydroxybenzaldéhydes de formule (VI) obtenus par un procédé comprenant l'hydroxyméthylation des phénols de formule (VIII) en phase aqueuse en présence d'une base alcaline ou alcalino-terreuse en polyméthylolphénols de formule générale :

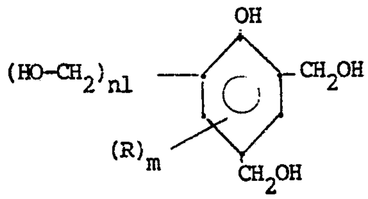

(X)

par le formaldéhyde ou un composé générateur de formaldéhyde, puis oxydation sans séparation en phase aqueuse alcaline, des polyméthylolphénols de formule (X) par l'oxygène moléculaire ou un gaz qui en contient en présence d'un catalyseur à base de palladium ou de platine contenant éventuellement un métal activateur usuel.

La combinaison du procédé d'oxydation des hydroxybenzaldéhydes conformes à la présente invention avec les deux étapes de préparation des hydroxybenzaldéhydes de départ par hydroxyméthylation d'un phénol par le formaldéhyde en phase aqueuse puis oxydation par l'oxygène en phase aqueuse alcaline en présence d'un catalyseur au platine ou au palladium est particulièrement avantageuse du point de vue industriel. En effet, en raison de l'analogie des milieux réactionnels de chacune des trois étapes il n'est pas nécessaire de séparer les produits intermédiaires à l'issue de chaque étape et l'on peut donc passer très simplement des phénols initiaux aux polyphénols ou aux polyhydroxybenzaldéhydes finals. Lors de la phase d'hydroxyméthylation du phénol de départ il peut se former suivant les conditions de réaction divers méthylolphénols différant entre eux par le nombre et/ou la position des groupes méthylols qu'il n'est pas nécessaire de séparer les uns des autres ; le mélange des différents mono et/ou polyméthylolphénols ainsi obtenu est ensuite soumis directement à l'oxydation par l'oxygène moléculaire pour donner naissance à un mélange de mono- ou polyformylphénols qui peuvent être oxydés directement par le procédé selon l'invention. Ainsi à partir du phénol on peut obtenir par hydroxyméthylation l'alcool orthohydroxybenzylique, l'alcool parahydroxybenzylique, le bis-hydroxyméthyl-2,6 phénol, le bis-hydroxyméthyl-2,4 phénol, le tri-hydroxyméthyl-2,4,6 phénol généralement sous forme de mélanges de deux ou plus de ces composés plus ou moins riches en chacun d'eux suivant les conditions de réaction. De la même façon, à partir du gaïacol on peut obtenir des mélanges contenant deux ou trois des hydroxyméthylméthoxyphénols suivants : le méthoxy-2 hydroxyméthyl-4 phénol, le méthoxy-2 hydroxyméthyl-6 phénol, le méthoxy-2 bis-hydroxyméthyl-4,6 phénol.

Par oxydation par l'oxygène moléculaire on obtiendra des mélanges d'hydroxybenzaldéhydes contenant deux ou plus des aldéhydes ci-après : salicylaldéhyde, p-hydroxybenzaldéhyde, diformyl-2,4 phénol, diformyl-2,6 phénol, triformyl-2,4,6 phénol au départ du phénol ou bien hydroxy-2 méthoxy-3 benzaldéhyde, vanilline et méthoxy-2 diformyl-4,6 phénol à partir du gaïacol.

Les conditions choisies pour le déroulement des étapes d'hydroxyméthylation et d'oxydation des méthylolphénols sont celles enseignées par l'art antérieur rappelé ci-avant.

En général l'étape d'hydroxyméthylation est conduite à une température comprise entre 0 et 100 °C et de préférence entre 20 et 70 °C ; le rapport molaire formaldéhyde/phénol gouverne la nature des hydroxyméthylphénols obtenus et par conséquent celle des polyphénols finals ; ce rapport peut varier entre 0,1/1 et 4 et de préférence entre 0,8 et 4.

La quantité de base présente dans le milieu d'hydroxyméthylation exprimée par le nombre de moles de base/hydroxyle phénolique du phénol à hydroxyméthyler peut varier dans de larges limites. En général ce rapport, variable suivant la nature de la base, peut varier entre 0,1 et 2 et de préférence entre 0,5 et 1,1. Comme base on peut utiliser celles citées plus haut pour la phase d'oxydation par l'eau oxygénée. L'emploi des hydroxydes alcalins (potasse et soude notamment) en solution aqueuse est particulièrement commode.

Le formaldéhyde peut être utilisé, sous forme de solution aqueuse dont la concentration n'est pas critique. Elle peut varier entre 20 et 50 % en poids ; on utilise de préférence les solutions commerciales dont la concentration est d'environ 30 à 40 % en poids.

Parmi les phénols de formule (VIII) qui peuvent servir de point de départ à la synthèse des polyphénols et plus spécialement des polyhydroxybenzaldéhydes on peut citer le phénol, l'hydroquinone, la pyrocatéchine, le gaïacol, l'éthoxy-2 phénol, le propoxy-2 ortho-crésol, le méthoxy-3 phénol, l'éthoxy-3 phénol, l'isopropoxyl-3 phénol, le t-butyloxy-3 phénol, le m-crésol, l'ortho-crésol, le para-crésol.

L'oxydation des méthylphénols en hydroxybenzaldéhydes intermédiaires par l'oxygène moléculaire peut être conduite comme cela a été indiqué plus haut directement sur la solution aqueuse alcaline des sels des méthylolphénols obtenus à l'étape d'hydroxyméthylation. Si cela est nécessaire le pH de la solution est porté à une valeur comprise entre 8 et 13 par addition éventuelle d'une base alcaline ou alcalino-terreuse. La valeur optimale du pH dépend de la nature des méthylolphénols.

Les catalyseurs mis en œuvre au cours de cette étape d'oxydation peuvent être choisis parmi ceux décrits dans les brevets américain 3.673.257, français N° 75.09932 et la demande de brevet français N° 77.13941 précités. On utilise de préférence des catalyseurs au platine et/ou au palladium pris sous toutes les formes disponibles (noir de platine, noir de palladium, platine ou palladium métallique) déposés de préférence sur un support inerte tel que le noir de carbone, les charbons actifs, la silice, l'amiante, l'alumine. L'activateur peut être choisi parmi tous ceux signalés dans les brevets ou demandes de brevets précités. De préférence on fait appel au bismuth, au plomb et au cadmium sous forme de métaux libres ou

de cations. Dans ce dernier cas l'anion associé n'est pas critique et on peut donc utiliser tous dérivés de ces métaux. De préférence on met en œuvre le bismuth métal ou ses dérivés.

On peut citer en particulier : les oxydes de bismuth ; les hydroxydes de bismuth ; les sels d'hydracides minéraux tels que : chlorure, bromure, iodure, sulfure, séléniure, tellurure de bismuth ; les sels d'oxyacides minéraux tels que : sulfite, sulfate, nitrite, nitrate, phosphite, phosphate, pyrophosphate, carbonate, perchlorate, antimoniate, arséniate, sélénite, séléniate de bismuth ; les sels d'oxyacides dérivés de métaux de transition tels que : vanadate, niobate, tantalate, chromate, molybdate, tungstate, permanganate de bismuth.

D'autres composés appropriés sont également des sels d'acides organiques aliphatiques ou aromatiques tels que : acétate, propionate, benzoate, salicylate, oxalate, tartrate, lactate, citrate de bismuth ; des phénates tels que : gallate et pyrogallate de bismuth. Ces sels et phénates peuvent être aussi des sels de bismuthyle.

Comme autres composés minéraux ou organiques, on peut utiliser des combinaisons binaires du bismuth avec des éléments tels que phosphore et arsenic ; des hétéropolyacides contenant du bismuth ainsi que leurs sels ; conviennent également les bismuthines aliphatiques et aromatiques.

A titre d'exemples spécifiques on peut citer :

— comme oxydes : BiO ; $Bi_2O_3$ ; $Bi_2O_4$ ; $Bi_2O_5$,

— comme hydroxydes : $Bi(OH)_3$.

— comme sels d'hydracides minéraux : le chlorure de bismuth $BiCl_3$ ; le bromure de bismuth $BiBr_3$ ; l'iodure de bismuth $BiI_3$ ; le sulfure de bismuth $Bi_2S_3$ ; le séléniure de bismuth $Bi_2Se_3$ ; le tellurure de bismuth $Bi_2Te_3$.

— comme sels d'oxyacides minéraux : le sulfite basique de bismuth $Bi_2(SO_3)_3$, $Bi_2O_3$, 5 $H_2O$ ; le sulfate neutre de bismuth $Bi_2(SO_4)_3$ ; le sulfate de bismuthyle $(BiO)HSO_4$ ; le nitrite de bismuthyle $(BiO)NO_2$, 0,5 $H_2O$ ; le nitrate neutre de bismuth $Bi(NO_3)_3$, 5 $H_2O$ ; le nitrate double de bismuth et de magnésium 2 $Bi(NO_3)_3$, 3 $Mg(NO_3)_2$, 24 $H_2O$ ; le nitrate de bismuthyle $(BiO)NO_3$ ; le phosphite de bismuth $Bi_2(PO_3H)_3$, 3 $H_2O$ ; le phosphate neutre de bismuth $BiPO_4$ ; le pyrophosphate de bismuth $Bi_4(P_2O_7)_3$ ; le carbonate de bismuthyle $(BiO)_2CO_3$, 0,5 $H_2O$ ; le perchlorate neutre de bismuth $Bi(ClO_4)_3$, 5 $H_2O$ ; l'antimoniate de bismuth $BiSbO_4$ ; l'arséniate neutre de bismuth $Bi(AsO_4)_3$ ; l'arséniate de bismuthyle $(BiO)AsO_4$, 5 $H_2O$ ; le sélénite de bismuth $Bi_2(SeO_3)_3$ ; le vanadate de bismuth $BiVO_4$ ; le niobate de bismuth $BiNbO_4$ ; le tantalate de bismuth $BiTaO_4$ ; le chromate neutre de bismuth $Bi_2(CrO_4)_3$, 3,5 $H_2O$ ; le chromate neutre de bismuthyle $(BiO)_2CrO_4$ ; le dichromate de bismuthyle $(BiO)_2Cr_2O_7$ ; le chromate acide de bismuthyle $H(BiO)CrO_4$ ; le chromate double de bismuthyle et de potassium $K(BiO)Cr_3O_{10}$ ; le molybdate de bismuth $Bi_2(MoO_4)_3$ ; le tungstate de bismuth $Bi_2(WO_4)_3$ ; le molybdate double de bismuth et de sodium $NaBi(MoO_4)_2$ ; le permanganate basique de bismuth $Bi_2O_2(OH)MnO_4$.

— comme sels d'acides organiques aliphatiques ou aromatiques : l'acétate de bismuth $Bi(C_2H_3O_2)_3$ ; le propionate de bismuthyle $(BiO)C_3H_5O_2$ ; le benzoate basique de bismuth $C_6H_5CO_2Bi(OH)_2$ ; le salicylate de bismuthyle $C_6H_4CO_2(BiO)(OH)$ ; l'oxalate de bismuth $(C_2O_4)_3Bi_2$ ; le tartrate de bismuth $Bi_2(C_4H_4O_6)_3$, 6 $H_2O$ ; le lactate de bismuth $(C_6H_9O_5)OBi$, 7 $H_2O$ ; le citrate de bismuth $C_6H_5O_7Bi$.

— comme phénates : le gallate basique de bismuth $C_7H_7O_7Bi$ ; le pyrogallate basique de bismuth $C_6H_3(OH)_2(OBi)(OH)$.

Comme autres composés minéraux ou organiques conviennent également : le phosphure de bismuth ; l'arséniure de bismuth $Bi_3As_4$ ; le bismuthate de sodium $NaBiO_3$ ; les acides bismuth-thiocyaniques $H_2[Bi(CNS)_5]$, $H_3[Bi(CNS)_6]$ et leurs sels de sodium et potassium ; la triméthylbismuthine $Bi(CH_3)_3$, la triphénylbismuthine $Bi(C_6H_5)_3$.

Les dérivés du bismuth qui sont utilisés de préférence sont : les oxydes de bismuth ; les hydroxydes de bismuth ; les sels de bismuth ou de bismuthyle d'hydracides minéraux ; les sels de bismuth ou de bismuthyle d'oxyacides minéraux ; les sels de bismuth ou de bismuthyle d'acides organiques aliphatiques ou aromatiques ; et les phénates de bismuth ou de bismuthyle.

Un groupe d'activateurs qui conviennent particulièrement bien est constitué par : le bismuth métallique ; les oxydes de bismuth $Bi_2O_3$ et $Bi_2O_4$ ; l'hydroxyde de bismuth $Bi(OH)_3$ ; le sulfate neutre de bismuth $Bi_2(SO_4)_3$ ; le chlorure de bismuth $BiCl_3$ ; le bromure de bismuth $BiBr_3$ ; l'iodure de bismuth $BiI_3$ ; le nitrate neutre de bismuth $Bi(NO_3)_3$, 5 $H_2O$ ; le carbonate de bismuthyle $(BiO)_2CO_3$, 0,5 $H_2O$ ; l'acétate de bismuth $Bi(C_2H_5O_2)_3$ ; le salicylate de bismuthyle $C_6H_4CO_2(BiO)(OH)$.

La quantité d'activateur utilisée, exprimée par la quantité de métal contenue dans l'activateur par rapport au poids du métal noble engagé, peut varier dans de larges limites. Par exemple, cette quantité peut être aussi petite que 0,1 % et peut atteindre le poids de métal noble engagé et même le dépasser sans inconvénient.

La quantité de catalyseur à mettre en œuvre, exprimée en poids de platine ou de palladium métallique par rapport à celui de l'hydroxyméthylphénol, peut varier de 0,01 à 4 % et de préférence de 0,04 à 2 %.

La concentration pondérale de l'hydroxyméthylphénol dans le milieu aqueux est habituellement comprise entre 1 % et 60 %, de préférence entre 2 % et 30 %.

Lorsqu'on prépare les polyphénols à partir d'un phénol avec enchaînement des étapes d'hydroxyméthylation, d'oxydation par l'oxygène moléculaire puis par l'eau oxygénée, il est préférable pour des raisons de commodité d'utiliser la même base pour amener le pH à la valeur désirée à chaque étape. De

préférence on fait appel à une base alcaline et en particulier aux hydroxydes de sodium et de potassium.

Pratiquement une manière d'exécuter l'oxydation de l'hydroxyméthylphénol consiste à mettre en contact avec de l'oxygène moléculaire, ou un gaz en contenant la solution aqueuse renfermant le composé à oxyder, l'agent alcalin, le catalyseur à base de métal noble et l'activateur. On opère à pression atmosphérique, mais on peut aussi le cas échéant opérer sous pression. Le mélange est ensuite agité à la température désirée jusqu'à consommation d'une quantité d'oxygène correspondant à celle nécessaire, dans les conditions de la réaction pour transformer l'hydroxyméthylphénol en hydroxybenzaldéhyde. D'une façon générale l'oxydation est conduite à une température de l'ordre de 10 à 100 °C, de préférence de 20 à 60 °C. Après refroidissement on sépare, s'il y a lieu, le catalyseur de la masse réactionnelle par exemple par filtration puis on porte le pH de la masse réactionnelle à une valeur inférieure à 7 par addition d'une quantité adéquate d'un acide minéral. L'oxydation par l'eau oxygénée est alors conduite comme il a été dit plus haut.

Le procédé décrit ci-avant enchaînant les phases d'hydroxyméthylation, d'oxydation par l'oxygène et de DAKIN en milieu acide convient tout particulièrement bien à la préparation de l'hydroxy p-vanilline à partir du gaïacol. Un tel procédé constitue un autre objet de la présente invention.

Dans ce cas l'étape d'hydroxyméthylation est conduite à une température comprise de préférence entre 20 et 60 °C. On pourrait sortir de ces limites mais sans que cela apporte d'avantages particuliers. Le rapport formol/gaïacol est généralement choisi au voisinage de la stœchiométrie, c'est-à-dire au voisinage de 2 ; des valeurs comprises entre 2 et 4 conviennent bien. De préférence on fait appel à des rapports formol/gaïacol allant de 2 à 2,7.

Bien que l'on puisse utiliser toute base alcaline ou alcalino-terreuse au cours de l'hydroxyméthylation du gaïacol, on met en œuvre de préférence un hydroxyde alcalin tel que la soude ou la potasse. Le rapport molaire base alcaline/gaïacol est voisin de la quantité nécessaire à la neutralisation de la fonction phénol, c'est-à-dire voisin de 1. On peut toutefois opérer avec un défaut de base ou un léger excès sans que cela se traduise par des inconvénients majeurs. Un rapport molaire base/gaïacol compris entre 0,5 et 1,2 convient tout particulièrement bien. La concentration des réactifs dans le milieu réactionnel n'est pas critique et peut varier dans de larges limites. Les conditions d'oxydation du dihydroxyméthylgaïacol en diformylgaïacol par l'oxygène en présence d'un métal noble sont celles définies généralement ci-avant. En pratique la température de la réaction est choisie entre 30 et 60 °C. Le pH du milieu d'oxydation du dihydroxyméthylgaïacol est de préférence au moins égal à 11 et plus préférentiellement il est compris entre 11,5 et 12. La concentration du dihydroxyméthylgaïacolate dans la phase aqueuse peut varier dans de larges limites. Elle est de préférence calculée pour ne pas dépasser les limites de solubilité du diformylgaïacolate formé. Les phases de condensation et d'oxydation peuvent être réalisées dans le même appareillage après avoir ajusté le pH à la valeur désirée et ajouté le catalyseur choisi. Pour réaliser l'étape suivante d'oxydation du diformylgaïacol en hydroxy p-vanilline, le catalyseur est séparé de la solution aqueuse alcaline de diformylgaïacolate par décantation ou filtration puis le pH du milieu est ajusté à la valeur désirée qui est comprise de préférence entre 4 et 5 par addition d'un acide fort. L'oxydation par l'eau oxygénée est ensuite conduite dans les conditions générales définies plus haut.

Bien que l'on puisse enchaîner directement les phases d'oxydation par l'oxygène et par l'eau oxygénée après avoir simplement procédé à un ajustage du pH, on a constaté qu'il est préférable de réaliser la précipitation du diformylgaïacol par acidification de la solution alcaline à pH 3 par addition d'un acide fort tel que l'acide sulfurique puis de séparer le précipité de la phase aqueuse acide par décantation et soutirage ou filtration. Le diformylgaïacol brut ainsi obtenu est alors mis en dispersion dans de l'eau et le pH de la suspension porté à la valeur désirée.

Le procédé enchaînant les phases d'hydroxyméthylation, d'oxydation par l'oxygène et de DAKIN en milieu acide convient également tout particulièrement bien pour la préparation du trihydroxy-3,4,5 benzaldéhyde, intermédiaire de la synthèse du triméthoxy-3,4,5 benzaldéhyde à partir du phénol. Un tel procédé, qui constitue un autre objet de la présente invention, met en œuvre l'hydroxyméthylation du phénol en triméthylol-2,4,6 phénol, l'oxydation par l'oxygène du triméthylolphénol en triformyl-2,4,6 phénol en milieu alcalin puis l'oxydation de ce dernier par l'eau oxygénée à pH inférieur ou égal à 7 en trihydroxy-3,4,5 benzaldéhyde. Comme cela a été indiqué précédemment le triformyl-2,4,6 phénol est un produit nouveau et à ce titre il constitue un autre objet de la présente invention.

Dans tous les cas, il est préférable de conduire les étapes de condensation et d'oxydation par l'eau oxygénée sous atmosphère d'un gaz inerte tel que l'argon ou l'azote.

Les exemples suivants illustrent l'invention et montrent comment elle peut être mise en pratique.

## Exemple 1

Dans un ballon en verre de 100 $cm^3$, équipé d'un agitateur, de deux ampoules de coulée, d'un thermomètre, d'un dispositif de chauffage, d'une arrivée de gaz inerte et d'une électrode en verre pour le contrôle du pH, on charge, sous atmosphère d'argon, 36 ml d'eau, 3,30 g (18 mM) de diformylgaïacol à 98 % puis on ajoute le pH à 4 par addition d'une solution aqueuse de soude à 15 % en poids présente dans l'une des deux ampoules de coulée. On porte le contenu du ballon à 55 °C et ajoute progressivement 2,53 g d'une solution aqueuse à 31,5 % en poids d'$H_2O_2$ (23,4 mM) de façon à maintenir la température à 50-55 °C. Au fur et à mesure du déroulement de la réaction, on ajoute progressivement la solution de soude

pour maintenir le pH à sa valeur de 4. L'addition de l'eau oxygénée est achevée en 30 mn ; on maintient la masse réactionnelle encore 10 mn à 50 °C puis la refroidit à 20 °C et dose les produits présents dans la phase aqueuse par chromatographie liquide haute pression.

On a obtenu les résultats suivants :
— diformylgaïacol : 0,13 g (taux de transformation 96 %)
— rendement en hydroxy p-vanilline par rapport au diformylgaïacol transformé : 94 %
— rendement en hydroxy o-vanilline par rapport au diformylgaïacol transformé : 6 %.

Exemples 2 à 5

On opère comme à l'exemple 1 mais en maintenant le pH du milieu réactionnel aux valeurs indiquées ci-après par mise en œuvre de la quantité adéquate de soude. On a obtenu les résultats consignés dans le tableau ci-après.

| EX. | pH | TT DFG (1) % | RT en HPV (2) % | Durée de réaction |
|---|---|---|---|---|
| 2 | 7 | 90 | 70 | 40 mn |
| 3 | 6 | 95 | 77 | 40 mn |
| 4 | 4,7 | 96 | 90 | 40 mn |
| 5 | 3,2 | 76 | 92 | 1 h |

(1) taux de transformation du diformylgaïacol
(2) rendement en hydroxy p-vanilline par rapport au diformylgaïacol transformé.

A titre comparatif on a répété l'exemple 1 en maintenant le pH à des valeurs supérieures à 7 ; on a obtenu les résultats suivants :

| ESSAIS | pH | TT DFG | RT HPV |
|---|---|---|---|
| A | 8 | 80 | 50 |
| B | 10 | 75 | 45 |
| C | 11 | 56 | 35 |
| D | 12 | 27 | 18,5 |

Exemple 6

Dans l'appareillage décrit à l'exemple 1 et en opérant de la même façon on oxyde 20 mM de diformylgaïacol à 98 % dans 40 ml d'eau, à 45 °C, par 26 mM de $H_2O_2$ en solution aqueuse à 31,5 % en maintenant le pH à 6,75 par addition d'une solution aqueuse de carbonate de sodium à 25 % en poids (au total on a ajouté 16 mM de $Na_2CO_3$). Puis on dose les produits présents dans la masse réactionnelle par chromatographie liquide haute pression. Le taux de transformation du diformylgaïacol est de 89 % et le rendement en hydroxy p-vanilline par rapport au diformylgaïacol transformé est de 78 %.

Exemple 7

On a opéré comme à l'exemple 6 mais en chargeant 20 mM de $H_2O_2$ au lieu de 26 mM. Le taux de transformation du diformylgaïacol est alors de 73 % et le rendement en hydroxyparavanilline par rapport au diformylgaïacol transformé de 88 %.

Exemple 8

Dans l'appareil décrit à l'exemple 1 purgé à l'argon, on charge :
— 50 ml d'eau désaérée
— 6,91 g (50 mM) d'aldéhyde protocatéchique à 97 %,

puis on ajuste le pH à 6 par addition d'une solution aqueuse de soude à 30 % en poids et porte la température à 45 °C. On ajoute alors au goutte à goutte, en 1 heure, 5,4 g d'eau oxygénée à 31,5 % en poids (50 mM) sous atmosphère d'argon. Le pH et la température sont maintenus aux valeurs indiquées ci-avant. On poursuit l'agitation pendant 30 mn après la fin de la coulée puis on prélève un échantillon de la masse réactionnelle sur lequel on dose les produits présents par chromatographie liquide haute pression. On ajoute alors au goutte à goutte une quantité supplémentaire de 2,6 g d'eau oxygénée (24 mM) et de la soude à 30 % en poids pour maintenir le pH et la température aux valeurs initiales. On procède ensuite à un nouveau dosage des produits présents. Les quantités de soude utilisées dans chacune des deux phases de l'expérience ont été respectivement de 5,2 g et 1,2 g.

A titre comparatif on a répété la même expérience à pH = 8 et pH = 9. Les résultats obtenus sont consignés dans le tableau suivant :

| EX. | PH | $H_2O_2$ % de la théorie | SOUDE à 30 % : g | DUREE | APC * : g | APC * : TT % * | THB-1,2,4 * : g | THB-1,2,4 * : RT % * |
|---|---|---|---|---|---|---|---|---|
| 8 | 6 | 100 | 5,20 | 1 h30 | 1,8 | 74 | 3,22 | 69 |
| | | 150 | 1,2 | 1 h | 0,52 | 92,5 | 3,59 | 61,5 |
| ESSAI: A | 8 | 100 | 6,9 | 1 h | 1,8 | 74 | 1,61 | 34,5 |
| | | 150 | 2,2 | 45 mn | 0,28 | 96 | 1,70 | 28 |
| B | 9 | 100 | 7,8 | 45 mn | 2,7 | 61 | 0 | 0 |
| | | 150 | 2,7 | 30 mn | 1,8 | 74 | 0 | 0 |

* APC = aldéhyde protocatéchique.
* THB-1,2,4 = trihydroxy-1,2,4 benzène
* TT = taux de transformation
* RT = rendement par rapport à l'aldéhyde transformé.

## Exemple 9

Dans l'appareillage décrit à l'exemple 1 et purgé à l'argon on charge :

— 40 ml d'eau désaérée,
— 4,56 g (32 mM) de dihydroxy-2,3 benzaldéhyde à 97 % en poids,

puis on ajuste le pH à 6 par addition d'une solution aqueuse de soude à 30 % en poids. On place le contenu du ballon sous atmosphère d'argon puis le porte à 45 °C et ajoute progressivement sous agitation 3,45 g d'une solution aqueuse à 31,5 % en poids d'eau oxygénée (32 mM) de façon à maintenir la température à sa valeur initiale. Le pH est maintenu à la valeur de 6 par addition simultanée de soude à 30 %.

L'addition de l'eau oxygénée est achevée en 35 mn. On maintient la masse réactionnelle encore 30 mn à 45 °C puis la refroidit à 30 °C et dose les produits présents dans la phase aqueuse par chromatographie liquide haute pression. Au total on a ajouté 3,2 g de solution aqueuse de soude à 30 % en poids. La température de la masse réactionnelle est alors portée à nouveau à 45 °C, puis on ajoute une quantité d'eau oxygénée à 31,5 % de 1,6 g (15 mM) en maintenant le pH à la valeur de 6 par addition de soude à 30 % en poids, puis on opère comme précédemment.

A titre comparatif, on a réalisé les mêmes essais, mais en portant le pH A) à 8, B) à 10. Les résultats obtenus sont consignés dans le tableau ci-après.

| EX. | pH | $H_2O_2$ % théorie | Dihydroxy-2,3 benzaldéhyde g | Dihydroxy-2,3 benzaldéhyde TT % | pyrogallol g | pyrogallol RT % |
|---|---|---|---|---|---|---|
| 9 | 6 | 100 | 0,66 | 85 | 3,4 | 99 |
| | | 150 | 0,04 | 99 | 4,04 | 100 |
| ESSAI | | | | | | |
| A | 8 | 100 | 0,26 | 94 | 2,6 | 69 |
| | | 150 | 0 | 100 | 2,6 | 64 |
| B | 10 | 100 | 0,67 | 85 | 0,025 | 0,7 |
| | | 150 | 0,05 | 99 | 0,015 | 0,4 |

Exemple 10

Dans l'appareillage décrit à l'exemple 1, purgé à l'argon, on charge :

— 4,14 g d'aldéhyde protocatéchique à 97 % (30 mM)
— 4,14 g de dihydroxy-2,3 benzaldéhyde à 97 % (30 mM)
— 60 ml d'eau désaérée.

Le pH est porté à 5 par addition d'une solution aqueuse de soude à 30 % en poids puis on élève la température du contenu du ballon à 45 °C sous atmosphère d'argon. On ajoute ensuite progressivement 5,18 g d'eau oxygénée à 31,5 % en poids (48 mM) sous forte agitation. La durée de l'addition est de 50 mn. On maintient l'agitation pendant 30 mn après la fin de la coulée de l'eau oxygénée puis on effectue un prélèvement d'échantillon de masse réactionnelle pour dosage des produits présents par chromatographie en phase liquide.

On poursuit la réaction en ajoutant 4,43 g d'eau oxygénée (41 mM) et de la soude en maintenant la température et le pH aux valeurs initiales. On procède à un nouveau prélèvement de masse réactionnelle pour dosage des produits présents par chromatographie en phase liquide.

A titre comparatif, on a répété le même essai à pH 9, en utilisant des rapports molaires $H_2O_2$/nombre total de groupes aldéhydes respectivement de 0,8 et 1,5.

Les résultats de ces différentes expériences sont consignés dans le tableau suivant :

| EX. | pH | $H_2O_2$ % théorie | APC * g | APC * TT | THB-1,2,4 * g | THB-1,2,4 * RT | DHBZ * g | DHBZ * TT | PY * g | PY * RT |
|---|---|---|---|---|---|---|---|---|---|---|
| 10 | 5 | 80 | 2,53 | 39 | 0,70 | 47,5 | 0,79 | 81 | 2,67 | 87 |
| | | 150 | 1,49 | 64 | 0,80 | 33 | 0,08 | 98 | 2,89 | 78 |
| ESSAI | | | | | | | | | | |
| A | 9 | 80 | 2,9 | 30 | 0,05 | 4 | 0,12 | 97 | 2,06 | 56 |
| | | 150 | 1,12 | 73 | 0,14 | 5 | 0 | 100 | 1,40 | 37 |

* APC = aldéhyde protocatéchique
* THB-1,2,4 = trihydroxy-1,2,4 benzène
* DHBZ = dihydroxy-2,3 benzaldéhyde
* PY = pyrogallol

Exemple 11

1. Préparation du dihydroxyméthylgaïacol.

Dans un réacteur en verre à 5 tubulures, équipé d'un système d'agitation, d'un réfrigérant ascendant, d'une ampoule de coulée et d'un thermomètre, on charge :

— 49 g d'une solution aqueuse à 11,2 % en poids de soude
— 0,14 mole de gaïacol sous atmosphère d'argon.

La température de la solution aqueuse de gaïacolate de sodium est maintenue à 40 °C et on ajoute alors goutte à goutte 0,35 mole de formaldéhyde sous forme d'une solution aqueuse à 30 % en poids et la masse réactionnelle est maintenue sous agitation pendant 4 h 30 mn dans ces conditions.

On prélève alors un échantillon du milieu pour dosage du formol par potentiométrie après oximation et du gaïacol et des produits formés par chromatographie liquide haute pression.

Les résultats obtenus sont les suivants :

Taux de transformation :

| | |
|---|---|
| — gaïacol | 100 % |
| — formol | 74 % |

Rendements sur le gaïacol :

| | |
|---|---|
| — dihydroxyméthyl-4,6 gaïacol | 80,5 % |
| — alcool orthovanillique (méthoxy-2 hydroxyméthyl-6 phénol) | 3 % |
| — alcool p-vanillique (méthoxy-2 hydroxyméthyl-4 phénol) | 1,5 % |
| — bis-(hydroxy, méthoxy, hydroxyméthylphényl) méthane | 15,5 % |

2. Préparation du diformylgaïacol.

Dans le réacteur décrit précédemment contenant la masse réactionnelle obtenue, équipé d'une électrode et d'une arrivée de gaz par tube plongeant relié à une réserve d'oxygène sous une pression de 1 bar et d'une arrivée de solution de soude couplée à un régulateur de pH, on charge 193 g d'eau distillée, 60 mg de sulfate de bismuth et 1 g d'un catalyseur à base de platine déposé sur noir de carbone (soit 50 mg de platine). La température du contenu du ballon est portée à 45 °C, puis on fait arriver l'oxygène en maintenant le pH à une valeur de 11,8 par addition de soude à 30 % en poids. Après 6 heures dans ces conditions, on constate que la réaction cesse.

On prélève une partie aliquote de la masse réactionnelle pour dosage des produits présents par chromatographie liquide haute pression.

On obtient les résultats suivants :

| | |
|---|---|
| — taux de transformation du dihydroxyméthyl-4,6 gaïacol | 100 % |
| — rendements rapportés au gaïacol chargé à la première étape : | |
| — diformyl-4,6 gaïacol (hydroxy-2 méthoxy-3 isophtalaldéhyde) | 74 % |
| — orthovanilline | 1,3 % |
| — paravanilline | 1,5 % |

Le rendement en diformylgaïacol rapporté au dihydroxyméthyl-4,6 gaïacol formé à l'étape précédente s'élève à 92 %.

3. Préparation de l'hydroxy-paravanilline.

La solution d'oxydation brute résultant de l'étape précédente est filtrée sous l'atmosphère d'azote pour éliminer le catalyseur. On recueille 300 ml de filtrat contenant :

— diformylgaïacol : 100 mM
— orthovanilline et paravanilline 2 mM.

Le filtrat est désoxygéné par passage d'azote puis on abaisse son pH de 11,8 à 4,3 par addition d'une solution aqueuse d'acide sulfurique à 95 % en poids. La température est alors ajustée à 50 °C puis on coule goutte à goutte sous atmosphère inerte et avec une forte agitation 14,2 g d'eau oxygénée à 30 % en poids (125 mM) préalablement désoxygéné en maintenant le pH à 4 par addition simultanée de soude à 30 % en poids (13,3 g) également désoxygénée. L'addition dure 2 h 30 mn.

Après la fin de la coulée d'eau oxygénée on maintient encore dans ces conditions pendant 30 mn. On dose les produits présents dans la masse réactionnelle par chromatographie en phase gazeuse. On a obtenu les résultats suivants :

| | |
|---|---|
| — taux de transformation du diformylgaïacol | 99,5 % |
| — rendement en hydroxy-paravanilline par rapport au diformylgaïacol chargé | 89 % |
| — rendement en hydroxy-orthovanilline par rapport au diformylgaïacol chargé | 3,5 %. |

## Exemple 12

Phase 1. Préparation du dihydroxyméthylgaïacol.

Dans un réacteur inoxydable de 2,5 l à double enveloppe pour circulation d'eau thermostatée à 50 °C, équipé d'un agitateur à double turbine (400 t/mn) et de chicanes, d'un réfrigérant ascendant, d'une arrivée de gaz inerte par tube plongeant, de deux ampoules de coulée, d'un thermomètre et d'un dispositif de contrôle du pH, purgé à l'azote, on introduit sous agitation :

— 221,2 g d'une solution aqueuse de soude à 30,5 % en poids,
— 1 000 g d'eau distillée.

La température du contenu du réacteur est portée à 50 °C et maintenue à cette valeur puis on charge 207,7 g de gaïacol (1,673 mole). On ajoute alors progressivement en 10 mn 340,1 g d'une solution aqueuse de formol à 30 % en poids tandis que l'on fait arriver un courant d'azote dans la masse réactionnelle. On maintient ces conditions pendant 4 h 30 mn après le début de l'addition du formaldéhyde. On obtient ainsi 1 768,8 g de masse réactionnelle dont on prélève une partie aliquote sous atmosphère d'azote pour dosage des produits présents par chromatographie liquide sous haute pression. On a obtenu les résultats suivants :

| | |
|---|---|
| — taux de transformation du gaïacol | 99 % |
| (on a dosé 2,07 g de gaïacol non transformé dans la masse réactionnelle) | |
| — rendement en dihydroxyméthylgaïacol | 76 % |
| — rendement en alcool paravanillique | 5 % |
| — rendement en alcool orthovanillique | 3 % |
| — rendement en bis(méthoxy-3 hydroxy-4 hydroxyméthyl-5 phényl) méthane | 14 % |

Phase 2. Préparation du diformylgaïacol.

On prélève sous atmosphère d'azote 1 263 g de la masse réactionnelle précédente que l'on introduit dans un réacteur en acier inoxydable de 3,2 l, résistant à la pression et maintenu sous atmosphère d'azote. Ce réacteur est équipé d'une double enveloppe pour circulation d'eau thermostatée à 40 °C, d'une arrivée d'azote, d'une arrivée d'air, d'un réfrigérant ascendant, d'un dispositif de contrôle du pH, d'un thermomètre, d'un dispositif d'agitation à double turbine (450 t/mn) et de chicanes et dans lequel on a déjà chargé : 1 340 g d'eau distillée, 2,12 g d'un catalyseur à base de platine et d'oxyde de bismuth déposé sur noir de carbone et contenant 6 % en poids de platine métallique et 2 % en poids de bismuth métallique. On porte le contenu du réacteur sous agitation et son pH à 12 par addition d'une solution aqueuse de soude à 30 % en poids à 40 °C. On arrête l'arrivée d'azote et introduit de l'air à un débit de 700 l/h (à température et pression normales) et sous une pression absolue de 3 bars. On maintient le pH de la masse réactionnelle à 12 par addition périodique de soude à 30 % en poids. Après 7 heures dans ces conditions, on arrête l'arrivée d'air, dégaze le réacteur et purge à l'azote. On prélève alors un échantillon de masse réactionnelle pour dosage des produits présents par chromatographie liquide haute pression. On a obtenu les résultats suivants :

| | |
|---|---|
| — taux de transformation dihydroxyméthylgaïacol : | 100 % |
| — rendement diformylgaïacol/gaïacol chargé à la phase 1 : | 69 % |
| — rendement diformylgaïacol/dihydroxyméthylgaïacol chargé à la phase 2 : | 91 % |
| — rendement hydroxyméthyl-p-vanilline/gaïacol chargé à la phase 1 : | 0,8 % |
| — rendement hydroxyméthyl-o-vanilline/gaïacol chargé à la phase 1 : | 0,7 % |
| — rendement p-vanilline/gaïacol chargé à la phase 1 : | 4 % |
| — rendement o-vanilline/gaïacol chargé à la phase 1 : | 2 % |

Phase 3. Préparation de l'hydroxy p-vanilline.

On a obtenu en fin de réaction 2 375 g de masse réactionnelle. On décante le contenu du réacteur pour séparer le catalyseur de la phase liquide et soutire cette dernière sous atmosphère d'azote. On en prélève une partie aliquote de 2 000 g que l'on introduit dans le réacteur utilisé à la première phase préalablement purgé à l'azote. On acidifie le contenu du réacteur par addition de 370,3 g d'acide sulfurique à 20 % en refroidissant par circulation d'eau à 20 °C dans la double enveloppe. On amène ainsi le pH de 12 à 3,5 en provoquant la précipitation du diformylgaïacol. La température du milieu est alors de 25 °C. On décante le milieu pour séparer le diformylgaïacol de la phase liquide et soutire 1 903 g de phase

aqueuse acide. On introduit alors sous agitation 694 g d'eau ; on laisse décanter à nouveau et élimine 477 g d'eau. On porte le contenu du réacteur à 50 °C puis ajuste le pH à 4,5 par addition de soude à 30 % en poids et coule progressivement 73 g d'une solution aqueuse d'eau oxygénée à 35 % en poids en 43 mn tout en maintenant le pH à sa valeur initiale. On maintient 1 h 30 mn dans ces conditions puis on porte le pH à 6,5 par addition de soude. On prélève un échantillon de masse réactionnelle pour dosage des produits présents par chromatographie liquide haute pression. On obtient les résultats suivants :

— rendement hydroxyparavanilline/diformylgaïacol chargé présent dans la partie aliquote de masse réactionnelle mise en œuvre dans cette dernière phase : 90 %.

Exemple 13

Phase 1. Préparation du triméthylol-2,4,6 phénol.

Dans une fiole conique en verre de 250 ml munie d'un agitateur magnétique, on mélange 100 g d'une solution de formol à 30 % dans l'eau (1,0 mole) et 23,5 g de phénol. On complète à 125 ml par environ 10 ml d'eau, puis ajoute sous agitation, 10,2 g de soude en pastilles (0,25 mole). On refroidit par un bain de glace de façon à ne pas dépasser 30 à 40 °C. Quand la température est redescendue à 25 °C, on purge à l'argon, coupe l'agitation et laisse reposer 24 heures la solution homogène à température ambiante (22 à 23 °C).

Après cette période, on ne dose plus que 315 millimoles de formol libre. Le mélange réactionnel est alors coulé avec agitation sur 800 ml d'isopropanol froid. On agite encore 10 mn le mélange hétérogène, puis filtre sur appareil de Büchner l'abondant précipité blanc rose. Ce dernier est rincé à l'isopropanol puis à l'éther. On sèche à l'étuve, à 40 °C sous 1 mm de mercure, et obtient 40 g d'une poudre dans laquelle on dose par R.M.N.

— 11,5 % en mole d'un diméthylol phénate de sodium
— 83 % en mole de triméthylol-2,4,6 phénate de sodium
— 5,5 % en mole de sel disodique d'un bis(diméthylolhydroxyphényl) méthane.

Phase 2. Oxydation des méthylols.

Dans un ballon de 500 ml à cinq cols, muni d'une agitation centrale, d'une électrode double de verre destinée à mesurer le pH, d'un thermomètre, d'une ampoule de coulée et d'une arrivée de gaz, on charge 23,4 g de triméthylolphénate de sodium brut fraîchement préparé, contenant 0,10 mole de substrat, et 400 ml d'eau. L'appareil est purgé à l'argon.

On ajoute 1 g de platine à 4,3 % en poids sur noir et 60 mg de sulfate de bismuth III. L'appareil est alors purgé à l'oxygène pur et relié à une réserve d'oxygène à pression atmosphérique ; l'agitation est portée à 1 050 t/mn.

On élève la température à 45 °C et en même temps le pH à 11,0 par coulée de soude à 30 % en poids (12 g soit 0,09 mole).

Après 1 h 15, 3,8 litres d'oxygène ont été absorbés, et on ne consomme plus d'oxygène pendant les 25 minutes suivantes (3,83 litres). On filtre à chaud le catalyseur sur un verre fritté de porosité 4, lave le solide à l'eau, et refroidit le filtrat vers 10 °C. On acidifie celui-ci avec précaution par de l'acide sulfurique ; un précipité commence à apparaître vers pH 8. On arrête l'addition d'acide lorsque le pH atteint 4 (17 ml de $H_2SO_4$ à 25 % en poids, ont été utilisés). Le solide jaune obtenu est filtré sur verre fritté de porosité 3, lavé avec 10 ml d'eau, essoré et séché à 30 °C sous 1 mm de mercure. On obtient ainsi 14,7 g de poudre jaune. Le rendement pondéral en brut contenant essentiellement du triformylphénol et des oligomères s'élève à 82 %.

A partir de ce produit brut, on isole le composé désiré et purifié, par chromatographie liquide préparative, avec un rendement de 18 % sur le triméthylol-2,4,6 phénol de départ soit 3,2 g.

Ce composé non décrit dans la littérature présente les caractéristiques suivantes :

— Point de fusion sous 760 mm de mercure : 206 °C
— pKa dans l'eau (force ionique : 0,001 ; 23 °C) = 5,8
— Spectre de Résonance Magnétique Nucléaire :
  — singulet (2 H) à Delta = 10,30 ppm
  — singulet (1 H) à Delta = 9,99 ppm
  — singulet (2 H) à Delta = 8,52 ppm
— Spectre Infra-Rouge
  — bande hydroxyle lié intramoléculaire à 3 100 $cm^{-1}$
  — bande carbonyle aldéhyde à 1 690 $cm^{-1}$
  — bande carbonyle aldéhyde lié à 1 665 $cm^{-1}$
  — bandes aromatiques à 1 595, 1 460 à 1 445 $cm^{-1}$
— Spectre de masse
  — M = 178
  — M—CO = 150

— M—CHO = 149
— M—CO—CHO = 121
— M—2CO—CHO = 93
— M—3CO—CHO = 65

Le triformyl-2,4,6 phénol est insoluble dans l'eau à 25 °C et a les solubilités suivantes à 25 °C dans les solvants organiques :

— éthanol : 0,5 g/l
— oxyde d'éthyle : 0,25 g/l
— benzène : 3 g/l

Phase 3. Oxydation du triformylphénol par l'eau oxygénée à pH 7.

Dans un réacteur en verre de 50 ml à cinq cols, muni d'un thermomètre, d'une électrode, d'un réfrigérant et de deux ampoules de coulée, on charge 450 mg de triformylphénol (2,5 mM). On ajoute 12,5 ml d'eau désaérée, met l'agitation magnétique en marche et élève la température à 45 °C, le tout sous azote.

Le pH est élevé à 4,5 par de la soude à 10 % en poids contenue dans la première ampoule, puis on coule en goutte à goutte en 10 mn 1,96 g d'eau oxygénée à 10 % en poids, depuis la deuxième ampoule (5,8 mM).

La solution, hétérogène au départ, s'homogénéise peu à peu. On maintient la masse réactionnelle à 45 °C :

— 50 mn à pH 4,5
— 1 h 45 à pH 5-5,5

Au total on a coulé 4,16 mM de soude.

Phase 4. Méthylation du trihydroxy-3,4,5 benzaldéhyde.

Afin de mesurer plus aisément le rendement en aldéhyde gallique obtenu lors de l'étape précédente, on méthyle l'ensemble des produits obtenus de la façon suivante :

Dans l'appareillage décrit précédemment, on remplace l'ampoule de coulée de soude 10 % en poids par une ampoule contenant de la soude dégazée à 30 % en poids. L'ampoule d'eau oxygénée est remplacée par une ampoule contenant du sulfate de diméthyle (DMS).

On élève le pH de la masse à 8 en conservant une température de 45°, et l'atmosphère d'azote. Puis on coule 4,75 g de DMS en 40 mn (37,5 mM) en maintenant le pH à 8-8,5. On arrête l'essai quand le pH se stabilise, soit après 1 h, la quantité de soude 30 % utilisée s'élevant alors à 4,2 g (31,5 mM).

Après refroidissement à 20 °C, on acidifie à pH 3,5 par 0,25 ml d'$H_2SO_4$ à 50 % en poids, et extrait la masse réactionnelle par trois fois 20 ml de dichloréthane. Les extraits organiques sont lavés par 10 ml d'eau, séchés sur sulfate de sodium, et analysés par chromatographie en phase gazeuse.

La méthylation des hydroxyles est totale, car on n'observe ni aldéhyde gallique, ni hydroxy-5 p-vanilline, ni seringaldéhyde et/ou son isomère.

— Rendement en triméthoxy-3,4,5 benzaldéhyde : 216 mg = 44 % par rapport au triformylphénol chargé à l'étape 3.

— Rendement en tétraméthoxy-1,3,4,5 benzène : 24 mg = 5 %.

— Le Rendement en aldéhyde gallique est donc d'au moins 44 % par rapport au triformyl-2,4,6 phénol chargé à l'étape 3.

**Revendications** (pour les Etats contractants : BE, CH, DE, FR, GB, IT, LI, LU, NL, SE)

1. Procédé de préparation de polyphénols comportant éventuellement un groupe aldéhyde par oxydation d'hydroxybenzaldéhydes comportant au moins un groupe aldéhyde en ortho et/ou para par rapport au groupe hydroxyle et répondant à la formule générale :

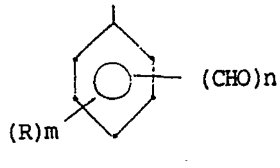

(I)

dans laquelle :

— n est un nombre entier de 1 à 3,

— R représente un radical alkyle, alkoxy, hydroxyalkyle, cycloalkyle, aryle, alkoxyalkyle, hydroxyle, un groupe nitro, un atome d'halogène,

— m est un nombre entier de 0 à 3, la somme m + n étant au plus égale à 4,

à l'exclusion des monohydroxybenzaldéhydes de formule (I) dans laquelle n est égal à 1, par l'eau oxygénée en milieu aqueux en présence d'une base alcaline ou alcalino-terreuse caractérisé en ce que le pH du milieu réactionnel est inférieur ou égal à 7 pendant la durée de la réaction.

2. Procédé de préparation de polyphénols selon la revendication 1, caractérisé en ce que les phénols obtenus répondent à la formule :

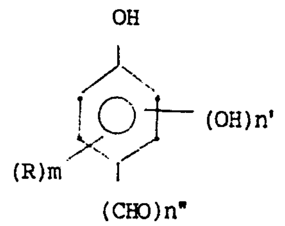

(II)

dans laquelle :

— R et m ont la signification déjà donnée,

— n' est un nombre entier de 1 à 2,

— n" est 0 ou 1, la somme n' + n" étant égale à n.

3. Procédé selon les revendications 1 et 2, caractérisé en ce que dans les formules (I) et (II) R représente un radical alkyle inférieur, un radical alcoxy inférieur, un groupe hydroxyle.

4. Procédé selon l'une quelconque des revendications 1 à 3 caractérisé en ce que n est égal à 2 ou 3.

5. Procédé selon l'une quelconque des revendications 1 à 4 caractérisé en ce que m est égal à 0 ou 1.

6. Procédé selon la revendication 1, caractérisé en ce que l'on prépare des polyhydroxybenzaldéhydes de formule générale :

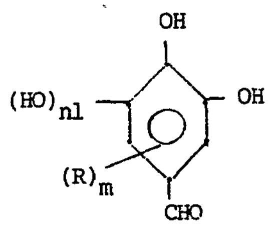

(VII)

dans laquelle R, m ont la signification donnée et $n_1$ est 0 ou 1 par oxydation d'un hydroxybenzaldéhyde de formule générale :

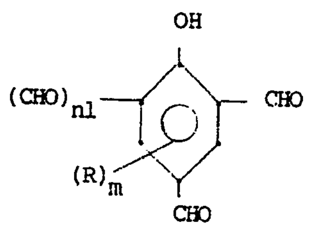

(VI)

7. Procédé selon la revendication 1, caractérisé en ce que l'on prépare du trihydroxy-1,2,4 benzène par oxydation de l'aldéhyde protocatéchique.

8. Procédé selon la revendication 1, caractérisé en ce que l'on prépare le pyrogallol par oxydation du dihydroxy-2,3 benzaldéhyde.

9. Procédé selon la revendication 1, caractérisé en ce que l'on prépare le pyrogallol et le trihydroxy-1, 2,4 benzène par oxydation d'un mélange d'aldéhyde protocatéchique et le dihydroxy-2,3 benzaldéhyde.

10. Procédé selon la revendication 1, caractérisé en ce que l'on prépare le dihydroxy-3,4 méthoxy-5 benzaldéhyde par oxydation du diformyl-4,6 gaïacol.

11. Procédé selon la revendication 1, caractérisé en ce que l'on prépare le trihydroxy-3,4,5 benzaldéhyde par oxydation du triformyl-2,4,6 phénol.

12. Procédé selon l'une quelconque des revendications 1 à 11, caractérisé en ce que la température de la réaction est comprise entre 0 et 100 °C.

13. Procédé selon l'une quelconque des revendications 1 à 12, caractérisé en ce que la quantité d'eau oxygénée exprimée en moles de $H_2O_2$ par groupe aldéhyde à oxyder est comprise entre 0,5 et 1,8.

14. Procédé selon l'une quelconque des revendications 1 à 13, caractérisé en ce que la base utilisée est un hydroxyde, un alcoolate ou un sel d'acide faible d'une base alcaline.

15. Procédé selon l'une quelconque des revendications 1 à 14 caractérisé en ce que la base alcaline est l'hydroxyde de sodium ou l'hydroxyde de potassium.

16. Procédé selon l'une quelconque des revendications 1 à 15, caractérisé en ce que la quantité de base est déterminée de façon à ce que le pH du milieu réactionnel reste dans les limites définies.

17. Procédé selon l'une quelconque des revendications 1 à 16, caractérisé en ce que le pH du milieu réactionnel est compris entre 2 et 6.

18. Procédé selon l'une quelconque des revendications 1 à 17, caractérisé en ce que l'hydroxybenzaldéhyde soumis à l'oxydation par l'eau oxygénée a été obtenu par un procédé en deux étapes comprenant l'hydroxyméthylation d'un phénol en mono et/ou polyméthylolphénols au moyen du formaldéhyde ou d'un générateur de formaldéhyde puis l'oxydation en phase aqueuse alcaline des mono- et/ou polyméthylolphénols par l'oxygène moléculaire ou un gaz qui en contient en présence d'un catalyseur à base d'un métal noble et éventuellement d'un activateur pris dans le groupe formé par Cd, Ce, Bi, Pb, Ag, Te et Sn ou leurs dérivés organiques ou minéraux.

19. Procédé selon la revendication 18, caractérisé en ce que l'on prépare des polyphénols de formule :

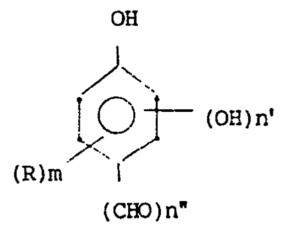

(II)

dans laquelle R, m, n' et n" ont la signification déjà indiquée, par hydroxyméthylation de phénols de formule :

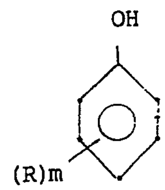

(VIII)

dans laquelle R et m ont la signification déjà donnée, en hydroxyméthylphénols de formule :

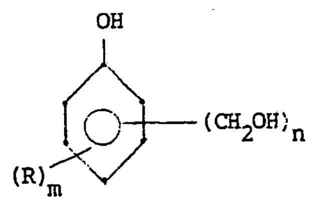

(IX)

dans laquelle R, m et n ont la signification déjà donnée, puis oxydation de ces derniers par l'oxygène moléculaire en phase aqueuse alcaline en hydroxybenzaldéhydes de formule :

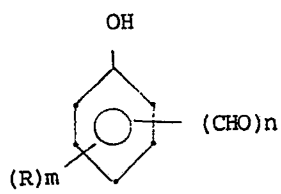

(I)

et enfin oxydation de ces derniers par l'eau oxygénée en phase aqueuse à pH ≤ 7.

20. Procédé selon la revendication 18, caractérisé en ce que l'on prépare des polyhydroxybenzaldéhydes de formule :

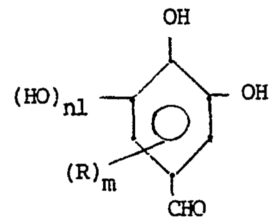

(VII)

dans laquelle R, m et $n_1$ ont la signification déjà donnée, par hydroxyméthylation d'un phénol de formule :

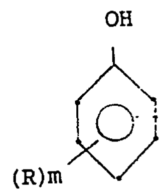

(VIII)

par le formaldéhyde ou un générateur de formaldéhyde en polyméthylolphénols de formule :

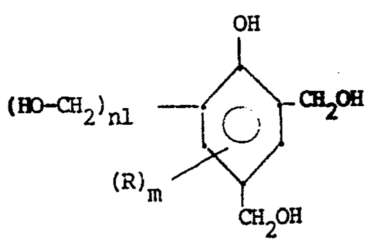

(X)

puis oxydation de ces derniers par l'oxygène moléculaire en phase aqueuse alcaline en aldéhydes de formule générale :

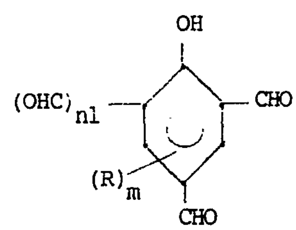

(VI)

et oxydation de ces derniers par l'eau oxygénée en phase aqueuse à pH ≤ 7 en présence d'une base alcaline ou alcalino-terreuse.

21. Procédé selon la revendication 18, caractérisé en ce que l'on prépare le trihydroxy-3,4,5 benzaldéhyde à partir du phénol par hydroxyméthylation de ce dernier en triméthylol-2,4,6 phénol, oxydation du triméthylolphénol par l'oxygène en triformyl-2,4,6 phénol et oxydation de ce dernier par l'eau oxygénée en phase aqueuse à pH ≤ 7 en présence d'une base alcaline ou alcalino-terreuse.

22. Procédé selon la revendication 18, caractérisé en ce que l'on prépare le dihydroxy-3,4 méthoxy-5 benzaldéhyde à partir du gaïacol par hydroxyméthylation de ce dernier en dihydroxyméthyl-4,6 méthoxy-2 phénol, oxydation du dihydroxyméthylgaïacol par l'oxygène moléculaire en diformylgaïacol et oxydation de ce dernier par l'eau oxygénée en phase aqueuse à pH ≤ 7 en présence d'une base alcaline ou alcalino-terreuse.

23. Procédé selon l'une quelconque des revendications 18 à 22, caractérisé en ce que l'hydroxyméthylation est réalisée en phase aqueuse en présence d'une base alcaline ou alcalino-terreuse, à une température comprise entre 0 et 100 °C, le rapport molaire formaldéhyde/phénol étant compris entre 0,1 et 4.

24. Procédé selon l'une quelconque des revendications 18 à 23, caractérisé en ce que l'oxydation des mono- et/ou polyméthylolphénols par l'oxygène moléculaire est réalisée en phase aqueuse alcaline à pH ≥ 8, à une température de 10 à 100 °C, en présence de 0,01 à 4 % en poids de platine ou de palladium métallique par rapport au méthylolphénol et éventuellement de 0,2 à 100 % en poids par rapport au métal noble d'un activateur.

25. Procédé selon l'une quelconque des revendications 18 à 24, caractérisé en ce que les étapes d'hydroxyméthylation en phase aqueuse, d'oxydation des mono- et/ou polyméthylolphénols par l'oxygène moléculaire en phase aqueuse alcaline et d'oxydation par l'eau oxygénée des hydroxybenzaldéhydes en phase aqueuse à pH ≤ 7 sont enchaînées sans séparation des composés intermédiaires formés au cours des deux premières étapes.

26. Procédé de préparation du dihydroxy-3,4 méthoxy-5 benzaldéhyde selon la revendication 22, caractérisé en ce que :

a) dans une première phase on prépare le dihydroxyméthylgaïacol par réaction d'une solution aqueuse de formaldéhyde ou d'un composé générateur de formaldéhyde avec une solution aqueuse d'un gaïacolate alcalin à une température comprise entre 20 et 60 °C, le rapport molaire formaldéhyde/gaïacol étant compris entre 2 et 4 et le rapport molaire base alcaline/gaïacol étant compris entre 0,5 et 1,2,

b) dans une deuxième phase on ajoute à la solution aqueuse de dihydroxyméthylgaïacolate alcalin obtenue à l'étape a) un catalyseur à base de platine déposé sur un support inerte et comportant éventuellement un activateur pris dans le groupe formé par Cd, Ce, Bi, Pb, Ag, Te et Sn ou leurs dérivés et procède à l'oxydation du dihydroxyméthylgaïacolate alcalin en diformylgaïacolate alcalin par l'oxygène moléculaire ou un gaz qui en contient, à une température comprise entre 30 et 60 °C, le pH du milieu étant compris entre 11,5 et 12, puis sépare le catalyseur,

c) dans une troisième phase on oxyde le diformylgaïacolate alcalin par addition progressive d'eau oxygénée à la solution aqueuse obtenue à la phase b) après avoir ajusté le pH à une valeur comprise entre 4 et 5 par addition d'un acide fort, la température étant comprise entre 30 et 80 °C et le pH maintenu entre 4 et 5 par coulée d'une base alcaline, le rapport molaire eau oxygénée/diformylgaïacolate alcalin étant compris entre 1 et 1,5.

27. Procédé selon la revendication 26, caractérisé en ce que les phases a) et c) sont conduites sous atmosphère d'un gaz inerte.

28. Procédé selon l'une quelconque des revendications 24 à 27, caractérisé en ce qu'après élimination du catalyseur à l'issue de la phase b) on acidifie la solution alcaline de diformylgaïacolate alcalin à pH inférieur ou égal à 3,5 pour précipiter le diformylgaïacol, on sépare ce dernier de la phase aqueuse puis le met en suspension dans l'eau, porte le pH à une valeur comprise entre 4 et 5 et procède à l'oxydation par l'eau oxygénée.

29. Nouveau polyformylphénol caractérisé en ce qu'il s'agit du triformyl-2,4,6 phénol.

**Revendications** (pour l'Etat contractant AT)

1. Procédé de préparation de polyphénols comportant éventuellement un groupe aldéhyde par oxydation d'hydroxybenzaldéhydes comportant au moins un groupe aldéhyde en ortho et/ou para par rapport au groupe hydroxyle et répondant à la formule générale :

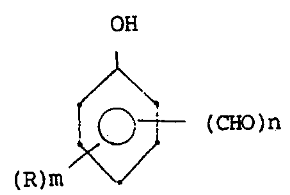

(I)

dans laquelle :

— n est un nombre entier de 1 à 3,

— R représente un radical alkyle, alkoxy, hydroxyalkyle, cycloalkyle, aryle, alkoxyalkyle, hydroxyle, un groupe nitro, un atome d'halogène,

— m est un nombre entier de 0 à 3, la somme m + n étant au plus égale à 4,

à l'exclusion des monohydroxybenzaldéhydes de formule (I) dans laquelle n est égal à 1, par l'eau oxygénée en milieu aqueux en présence d'une base alcaline ou alcalino-terreuse caractérisé en ce que le pH du milieu réactionnel est inférieur ou égal à 7 pendant la durée de la réaction.

2. Procédé de préparation de polyphénols selon la revendication 1, caractérisé en ce que les phénols obtenus répondent à la formule :

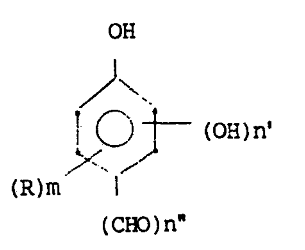

(II)

dans laquelle :

— R et m ont la signification déjà donnée,

— n' est un nombre entier de 1 à 2,

— n" est 0 ou 1, la somme n' + n" étant égale à n.

3. Procédé selon les revendications 1 et 2, caractérisé en ce que dans les formules (I) et (II) R représente un radical alkyle inférieur, un radical alcoxy inférieur, un groupe hydroxyle.

4. Procédé selon l'une quelconque des revendications 1 à 3 caractérisé en ce que n est égal à 2 ou 3.

5. Procédé selon l'une quelconque des revendications 1 à 4 caractérisé en ce que m est égal à 0 ou 1.

6. Procédé selon la revendication 1, caractérisé en ce que l'on prépare des polyhydroxybenzaldéhydes de formule générale :

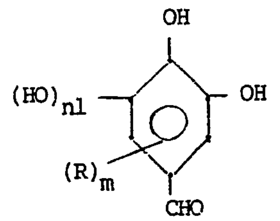

(VII)

dans laquelle R, m ont la signification donnée et $n_1$ est 0 ou 1 par oxydation d'un hydroxybenzaldéhyde de formule générale :

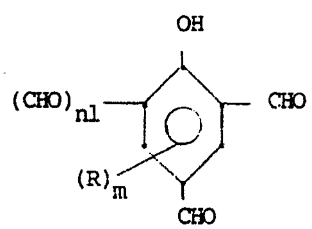

(VI)

7. Procédé selon la revendication 1, caractérisé en ce que l'on prépare du trihydroxy-1,2,4 benzène par oxydation de l'aldéhyde protocatéchique.

8. Procédé selon la revendication 1, caractérisé en ce que l'on prépare le pyrogallol par oxydation du dihydroxy-2,3 benzaldéhyde.

9. Procédé selon la revendication 1, caractérisé en ce que l'on prépare le pyrogallol et le trihydroxy-1,2,4 benzène par oxydation d'un mélange d'aldéhyde protocatéchique et le dihydroxy-2,3 benzaldéhyde.

10. Procédé selon la revendication 1, caractérisé en ce que l'on prépare le dihydroxy-3,4 méthoxy-5 benzaldéhyde par oxydation du diformyl-4,6 gaïacol.

11. Procédé selon la revendication 1, caractérisé en ce que l'on prépare le trihydroxy-3,4,5 benzaldéhyde par oxydation du triformyl-2,4,6 phénol.

12. Procédé selon l'une quelconque des revendications 1 à 11, caractérisé en ce que la température de la réaction est comprise entre 0 et 100 °C.

13. Procédé selon l'une quelconque des revendications 1 à 12, caractérisé en ce que la quantité d'eau oxygénée exprimée en moles de $H_2O_2$ par groupe aldéhyde à oxyder est comprise entre 0,5 et 1,8.

14. Procédé selon l'une quelconque des revendications 1 à 13, caractérisé en ce que la base utilisée est un hydroxyde, un alcoolate ou un sel d'acide faible d'une base alcaline.

15. Procédé selon l'une quelconque des revendications 1 à 14 caractérisé en ce que la base alcaline est l'hydroxyde de sodium ou l'hydroxyde de potassium.

16. Procédé selon l'une quelconque des revendications 1 à 15, caractérisé en ce que la quantité de base est déterminée de façon à ce que le pH du milieu réactionnel reste dans les limites définies.

17. Procédé selon l'une quelconque des revendications 1 à 16, caractérisé en ce que le pH du milieu réactionnel est compris entre 2 et 6.

18. Procédé selon l'une quelconque des revendications 1 à 17, caractérisé en ce que l'hydroxybenzaldéhyde soumis à l'oxydation par l'eau oxygénée a été obtenu par un procédé en deux étapes comprenant l'hydroxyméthylation d'un phénol en mono et/ou polyméthylolphénols au moyen du formaldéhyde ou d'un générateur de formaldéhyde puis l'oxydation en phase aqueuse alcaline des mono- et/ou polyméthylolphénols par l'oxygène moléculaire ou un gaz qui en contient en présence d'un catalyseur à base d'un métal noble et éventuellement d'un activateur pris dans le groupe formé par Cd, Ce, Bi, Pb, Ag, Te et Sn ou leurs dérivés organiques ou minéraux.

19. Procédé selon la revendication 18, caractérisé en ce que l'on prépare des polyphénols de formule :

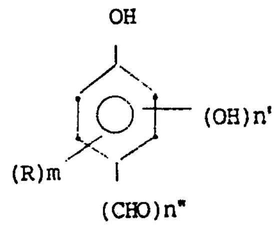

(II)

dans laquelle R, m, n' et n'' ont la signification déjà indiquée, par hydroxyméthylation de phénols de formule :

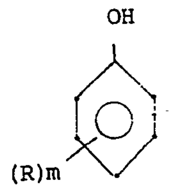

(VIII)

dans laquelle R et m ont la signification déjà donnée, en hydroxyméthylphénols de formule :

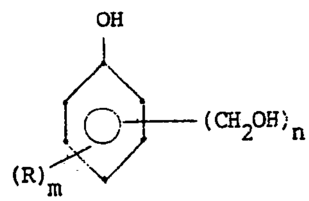

(IX)

dans laquelle R, m et n ont la signification déjà donnée, puis oxydation de ces derniers par l'oxygène moléculaire en phase aqueuse alcaline en hydroxybenzaldéhydes de formule :

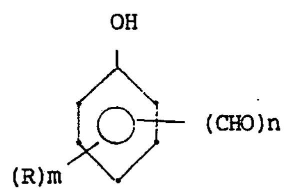

(I)

et enfin oxydation de ces derniers par l'eau oxygénée en phase aqueuse à pH ≤ 7.

20. Procédé selon la revendication 18, caractérisé en ce que l'on prépare des polyhydroxybenzaldéhydes de formule :

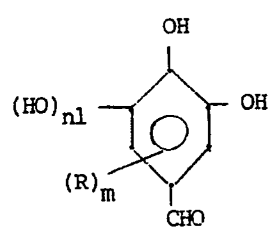

(VII)

dans laquelle R, m et $n_1$ ont la signification déjà donnée, par hydroxyméthylation d'un phénol de formule :

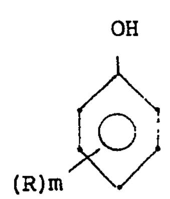

(VIII)

par le formaldéhyde ou un générateur de formaldéhyde en polyméthylolphénols de formule :

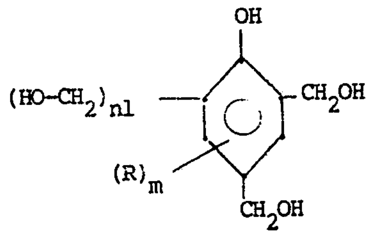

(X)

puis oxydation de ces derniers par l'oxygène moléculaire en phase aqueuse alcaline en aldéhydes de formule générale :

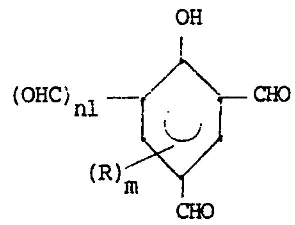

(VI)

et oxydation de ces derniers par l'eau oxygénée en phase aqueuse à pH ≤ 7 en présence d'une base alcaline ou alcalino-terreuse.

21. Procédé selon la revendication 18, caractérisé en ce que l'on prépare le trihydroxy-3,4,5 benzaldéhyde à partir du phénol par hydroxyméthylation de ce dernier en triméthylol-2,4,6 phénol, oxydation du triméthylolphénol par l'oxygène en triformyl-2,4,6 phénol et oxydation de ce dernier par l'eau oxygénée en phase aqueuse à pH ≤ 7 en présence d'une base alcaline ou alcalino-terreuse.

22. Procédé selon la revendication 18, caractérisé en ce que l'on prépare le dihydroxy-3,4 méthoxy-5 benzaldéhyde à partir du gaïacol par hydroxyméthylation de ce dernier en dihydroxyméthyl-4,6 méthoxy-2 phénol, oxydation du dihydroxyméthylgaïacol par l'oxygène moléculaire en diformylgaïacol et oxydation de ce dernier par l'eau oxygénée en phase aqueuse à pH ≤ 7 en présence d'une base alcaline ou alcalino-terreuse.

23. Procédé selon l'une quelconque des revendications 18 à 22, caractérisé en ce que l'hydroxyméthylation est réalisée en phase aqueuse en présence d'une base alcaline ou alcalino-terreuse, à une température comprise entre 0 et 100 °C, le rapport molaire formaldéhyde/phénol étant compris entre 0,1 et 4.

24. Procédé selon l'une quelconque des revendications 18 à 23, caractérisé en ce que l'oxydation des mono- et/ou polyméthylolphénols par l'oxygène moléculaire est réalisée en phase aqueuse alcaline à pH ≥ 8, à une température de 10 à 100 °C, en présence de 0,01 à 4 % en poids de platine ou de palladium métallique par rapport au méthylolphénol et éventuellement de 0,2 à 100 % en poids par rapport au métal noble d'un activateur.

25. Procédé selon l'une quelconque des revendications 18 à 24, caractérisé en ce que les étapes d'hydroxyméthylation en phase aqueuse, d'oxydation des mono- et/ou polyméthylolphénols par l'oxygène moléculaire en phase aqueuse alcaline et d'oxydation par l'eau oxygénée des hydroxybenzaldéhydes en phase aqueuse à pH ≤ 7 sont enchaînées sans séparation des composés intermédiaires formés au cours des deux premières étapes.

26. Procédé de préparation du dihydroxy-3,4 méthoxy-5 benzaldéhyde selon la revendication 22, caractérisé en ce que :

a) dans une première phase on prépare le dihydroxyméthylgaïacol par réaction d'une solution aqueuse de formaldéhyde ou d'un composé générateur de formaldéhyde avec une solution aqueuse d'un gaïacolate alcalin à une température comprise entre 20 et 60 °C, le rapport molaire formaldéhyde/gaïacol étant compris entre 2 et 4 et le rapport molaire base alcaline/gaïacol étant compris entre 0,5 et 1,2,

b) dans une deuxième phase on ajoute à la solution aqueuse de dihydroxyméthylgaïacolate alcalin obtenue à l'étape a) un catalyseur à base de platine déposé sur un support inerte et comportant éventuellement un activateur pris dans le groupe formé par Cd, Ce, Bi, Pb, Ag, Te et Sn ou leurs dérivés et procède à l'oxydation du dihydroxyméthylgaïacolate alcalin en diformylgaïacolate alcalin par l'oxygène moléculaire ou un gaz qui en contient, à une température comprise entre 30 et 60 °C, le pH du milieu étant compris entre 11,5 et 12, puis sépare le catalyseur.

c) dans une troisième phase on oxyde le diformylgaïacolate alcalin par addition progressive d'eau oxygénée à la solution aqueuse obtenue à la phase b) après avoir ajusté le pH à une valeur comprise entre 4 et 5 par addition d'un acide fort, la température étant comprise entre 30 et 80 °C et le pH maintenu entre 4 et 5 par coulée d'une base alcaline, le rapport molaire eau oxygénée/diformylgaïacolate alcalin étant compris entre 1 et 1,5.

27. Procédé selon la revendication 26, caractérisé en ce que les phases a) et c) sont conduites sous atmosphère d'un gaz inerte.

28. Procédé selon l'une quelconque des revendications 24 à 27, caractérisé en ce qu'après élimination du catalyseur à l'issue de la phase b) on acidifie la solution alcaline de diformylgaïacolate alcalin à pH inférieur ou égal à 3,5 pour précipiter le diformylgaïacol, on sépare ce dernier de la phase aqueuse puis le met en suspension dans l'eau, porte le pH à une valeur comprise entre 4 et 5 et procède à l'oxydation par l'eau oxygénée.

**Claims** (for the Contracting States : BE, CH, DE, FR, GB, IT, LI, LU, NL, SE)

1. Process for the preparation of polyphenols optionally containing an aldehyde group, by the oxidation of hydroxybenzaldehydes containing at least one aldehyde group in the ortho- and/or para-position to the hydroxyl group and corresponding to the general formula :

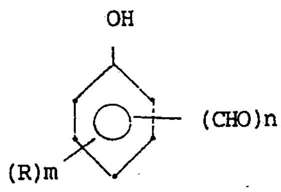

(I)

in which :

— n is an integer from 1 to 3,

— R represents an alkyl, alkoxy, hydroxyalkyl, cycloalkyl, aryl, alkoxyalkyl or hydroxyl radical, a nitro group or a halogen atom, and

— m is an integer from 0 to 3, the sum of m + n being equal to at most 4,

except for the monohydroxybenzaldehydes of the formula (I) in which n is equal to 1, with hydrogen peroxide, in an aqueous medium, in the presence of an alkali metal base or alkaline earth metal base, characterised in that the pH of the reaction medium is less than or equal to 7 throughout the reaction.

2. Process for the preparation of polyphenols according to Claim 1, characterised in that the phenols obtained correspond to the formula :

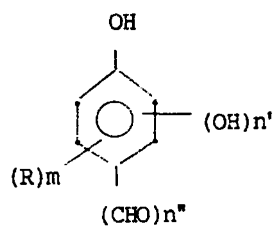

(II)

in which :

— R and m have the meanings already given,

— n' is an integer from 1 to 2, and

— n" is 0 or 1, the sum of n' + n" being equal to n.

3. Process according to Claims 1 and 2, characterised in that, in the formulae (I) and (II), R represents a lower alkyl radical, a lower alkoxy radical or a hydroxyl group.

4. Process according to any one of Claims 1 to 3, characterised in that n is equal to 2 or 3.

5. Process according to any one of Claims 1 to 4, characterised in that m is equal to 0 or 1.

6. Process according to Claim 1, characterised in that polyhydroxybenzaldehydes of the general formula :

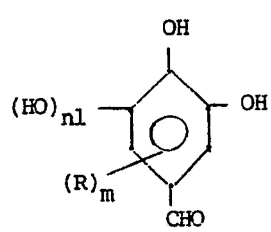

(VII)

in which R, m have the meanings given and $n_1$ is 0 or 1, are prepared by the oxidation of a hydroxybenzaldehyde of the general formula :

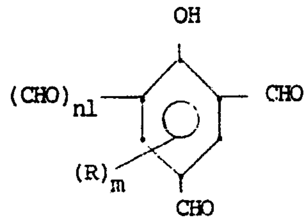

(VI)

7. Process according to Claim 1, characterised in that 1,2,4-trihydroxybenzene is prepared by the oxidation of protocatechualdehyde.

8. Process according to Claim 1, characterised in that pyrogallol is prepared by the oxidation of 2,3-dihydroxybenzaldehyde.

9. Process according to Claim 1, characterised in that pyrogallol and 1,2,4-trihydroxybenzene are prepared by the oxidation of a mixture of protocatechualdehyde and 2,3-dihydroxybenzaldehyde.

10. Process according to Claim 1, characterised in that 3,4-dihydroxy-5-methoxybenzaldehyde is prepared by the oxidation of 4,6-diformylguaiacol.

11. Process according to Claim 1, characterised in that 3,4,5-trihydroxybenzaldehyde is prepared by the oxidation of 2,4,6-triformylphenol.

12. Process according to any one of Claims 1 to 11, characterised in that the reaction temperature is between 0 and 100 °C.

13. Process according to any one of Claims 1 to 12, characterised in that the amount of hydrogen peroxide, expressed in mol of $H_2O_2$ per aldehyde group to be oxidised, is between 0.5 and 1.8.

14. Process according to any one of Claims 1 to 13, characterised in that the base used is a hydroxide, an alcoholate or a salt of a weak acid with an alkali metal base.

15. Process according to any one of Claims 1 to 14, characterised in that the alkali metal base in sodium hydroxide or potassium hydroxide.

16. Process according to any one of Claims 1 to 15, characterised in that the amount of base is determined so that the pH of the reaction medium remains within the defined limits.

17. Process according to any one of Claims 1 to 16, characterised in that the pH of the reaction medium is between 2 and 6.

18. Process according to any one of Claims 1 to 17, characterised in that the hydroxybenzaldehyde subjected to oxidation with hydrogen peroxide has been obtained by a two-step process comprising the hydroxymethylation of a phenol to mono- and/or poly-methylolphenols by means of formaldehyde or a formaldehyde generator, followed by the oxidation of the mono- and/or poly-methylolphenols with molecular oxygen or a gas which contains molecular oxygen, in an alkaline aqueous phase, in the presence of a catalyst based on a noble metal and, if appropriate, on an activator taken from the group comprising Cd, Ce, Bi, Pb, Ag, Te and Sn, or their organic or inorganic derivatives.

19. Process according to Claim 18, characterised in that polyphenols of the formula :

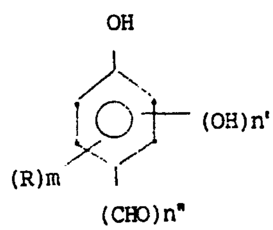

(II)

in which R, m, n' and n" have the meanings already indicated, are prepared by the hydroxymethylation of phenols of the formula :

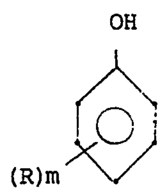

(VIII)

in which R and m have the meanings already given, to hydroxymethylphenols of the formula :

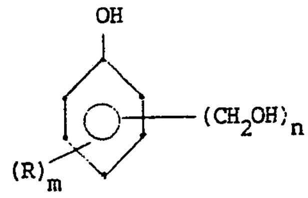

(IX)

in which R, m and n have the meanings already given, followed by the oxidation of the latter with molecular oxygen, in an alkaline aqueous phase, to hydroxybenzaldehydes of the formula :

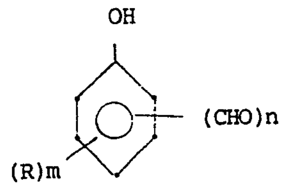

(I)

and finally the oxidation of the latter with hydrogen peroxide, in the aqueous phase, at pH $\leq$ 7.

20. Process according to Claim 18, characterised in that polyhydroxybenzaldehydes of the formula :

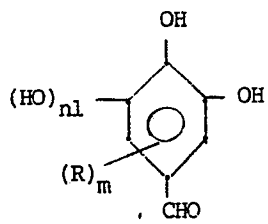

(VII)

in which R, m and $n_1$ have the meanings already given, are prepared by the hydroxymethylation of a phenol of the formula :

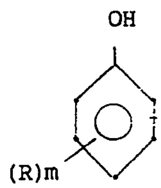

(VIII)

with formaldehyde or a formaldehyde generator, to polymethylolphenols of the formula :

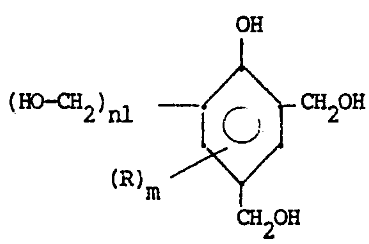

(X)

followed by oxidation of the latter with molecular oxygen, in an alkaline aqueous phase, to aldehydes of the general formula :

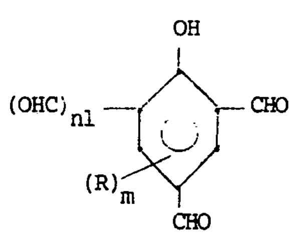

(VI)

and the oxidation of the latter with hydrogen peroxide, in the aqueous phase, at pH ≤ 7, in the presence of an alkali metal base or alkaline earth metal base.

21. Process according to Claim 18, characterised in that 3,4,5-trihydroxybenzaldehyde is prepared from phenol by the hydroxymethylation of the latter to 2,4,6-trimethylolphenol, the oxidation of the trimethylolphenol with oxygen to 2,4,6-triformylphenol, and the oxidation of the latter with hydrogen peroxide, in the aqueous phase, at pH ≤ 7, in the presence of an alkali metal base or alkaline earth metal base.

22. Process according to Claim 18, characterised in that 3,4-dihydroxy-5-methoxybenzaldehyde is prepared from guaiacol by the hydroxymethylation of the latter to 4,6-dihydroxymethyl-2-methoxyphenol, the oxidation of the dihydroxymethylguaiacol with molecular oxygen to diformylguaiacol, and the oxidation of the latter with hydrogen peroxide, in the aqueous phase, at pH ≤ 7, in the presence of an alkali metal base or alkaline earth metal base.

23. Process according to any one of Claims 18 to 22, characterised in that the hydroxymethylation is carried out in the aqueous phase, in the presence of an alkali metal base or alkaline earth metal base, at a temperature of between 0 and 100 °C, the molar ratio of formaldehyde/phenol being between 0.1 and 4.

24. Process according to any one of Claims 18 to 23, characterised in that the oxidation of the mono- and/or poly-methylolphenols with molecular oxygen is carried out in an alkaline aqueous phase, at pH ≤ 8, at a temperature of 10 to 100 °C, in the presence of 0.01 to 4 % by weight of platinum or palladium metal, relative to the methylolphenol, and, if appropriate, in the presence of 0.2 to 100 % by weight of an activator, relative to the noble metal.

25. Process according to any one of Claims 18 to 24, characterised in that the steps involving hydroxymethylation in the aqueous phase, oxidation of the mono- and/or poly-methylolphenols with molecular oxygen in an alkaline aqueous phase, and oxidation of the hydroxybenzaldehydes with hydrogen peroxide in the aqueous phase, at pH ≤ 7, are carried out in succession without separation of the intermediates formed during the first two steps.

26. Process for the preparation of 3,4-dihydroxy-5-methoxybenzaldehyde, according to Claim 22, characterised in that:

a) in a first stage, dihydroxymethylguaiacol is prepared by reacting an aqueous solution of formaldehyde, or of a compound which generates formaldehyde, with an aqueous solution of an alkali metal guaiacolate, at a temperature of between 20 and 60 °C, the molar ratio of formaldehyde/guaiacol being between 2 and 4 and the molar ratio of alkali metal base/guaiacol being between 0.5 and 1.2,

b) in a second stage, a platinum-based catalyst deposited on an inert support and, if appropriate, containing an activator taken from the group comprising Cd, Ce, Bi, Pb, Ag, Te and Sn, or their derivatives, is added to the aqueous solution of alkali metal dihydroxymethylguaiacolate obtained in step a), the alkali metal dihydroxymethylguaiacolate is oxidised to the alkali metal diformylguaiacolate with molecular oxygen or a gas which contains molecular oxygen, at a temperature of between 30 and 60 °C, the pH of the medium being between 11.5 and 12, and the catalyst is then separated off, and

c) in a third stage, the alkali metal diformylguaiacolate is oxidised by the gradual addition of hydrogen peroxide to the aqueous solution obtained in stage b), after adjustment of the pH to a value of between 4 and 5 by adding a strong acid, the temperature being between 30 and 80 °C and the pH being kept between 4 and 5 by running in an alkali metal base, and the molar ratio of hydrogen peroxide/alkali metal diformylguaiacolate being between 1 and 1.5.

27. Process according to Claim 26, characterised in that stages a) and c) are carried out under an inert gas atmosphere.

28. Process according to any one of Claims 24 to 27, characterised in that, after removal of the catalyst at the end of stage b), the alkaline solution of alkali metal diformylguaiacolate is acidified to a pH which is less than or equal to 3.5, in order to precipitate the diformylguaiacol, the latter is separated from the aqueous phase and then suspended in water, the pH is brought to a value of between 4 and 5 and oxidation is carried out with hydrogen peroxide.

29. New polyformylphenol, characterised in that it is 2,4,6-triformylphenol.

**Claims** (for the Contracting State AT)

1. Process for the preparation of polyphenols optionally containing an aldehyde group, by the oxidation of hydroxybenzaldehydes containing at least one aldehyde group in the ortho- and/or para-position to the hydroxyl group and corresponding to the general formula:

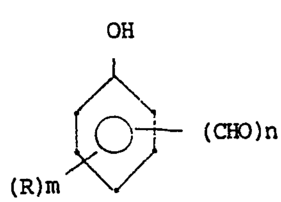

(I)

in which :

— n is an integer from 1 to 3,

— R represents an alkyl, alkoxy, hydroxyalkyl, cycloalkyl, aryl, alkoxyalkyl or hydroxyl radical, a nitro group or a halogen atom, and

— m is an integer from 0 to 3, the sum of m + n being equal to at most 4,

except for the monohydroxybenzaldehydes of the formula (I) in which n is equal to 1, with hydrogen peroxide, in an aqueous medium, in the presence or an alkali metal base or alkaline earth metal base, characterised in that the pH of the reaction medium is less than or equal to 7 throughout the reaction.

2. Process for the preparation of polyphenols according to Claim 1, characterised in that the phenols obtained correspond to the formula :

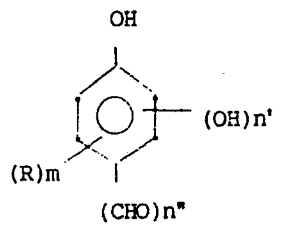

(II)

in which :

— R and m have the meanings already given,

— n′ is an integer from 1 to 2, and

— n″ is 0 or 1, the sum of n′ + n″ being equal to n.

3. Process according to Claims 1 and 2, characterised in that, in the formulae (I) and (II), R represents a lower alkyl radical, a lower alkoxy radical or a hydroxyl group.

4. Process according to any one of Claims 1 to 3, characterised in that n is equal to 2 or 3.

5. Process according to any one of Claims 1 to 4, characterised in that m is equal to 0 or 1.

6. Process according to Claim 1, characterised in that polyhydroxybenzaldehydes of the general formula :

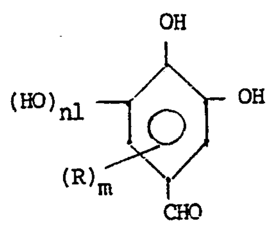

(VII)

in which R, m have the meanings given and $n_1$ is 0 or 1, are prepared by the oxidation of a hydroxybenzaldehyde of the general formula :

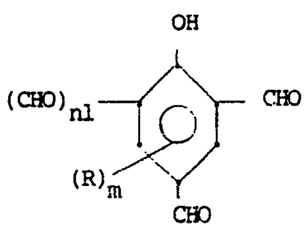

(VI)

7. Process according to Claim 1, characterised in that 1,2,4-trihydroxybenzene is prepared by the oxidation of protocatechualdehyde.

8. Process according to Claim 1, characterised in that pyrogallol is prepared by the oxidation of 2,3-dihydroxybenzaldehyde.

9. Process according to Claim 1, characterised in that pyrogallol and 1,2,4-trihydroxybenzene are prepared by the oxidation of a mixture of protocatechualdehyde and 2,3-dihydroxybenzaldehyde.

10. Process according to Claim 1, characterised in that 3,4-dihydroxy-5-methoxybenzaldehyde is prepared by the oxidation of 4,6-diformylguaiacol.

11. Process according to Claim 1, characterised in that 3,4,5-trihydroxybenzaldehyde is prepared by the oxidation of 2,4,6-triformylphenol.

12. Process according to any one of Claims 1 to 11, characterised in that the reaction temperature is between 0 and 100 °C.

13. Process according to any one of Claims 1 to 12, characterised in that the amount of hydrogen peroxide, expressed in mol of $H_2O_2$ per aldehyde group to be oxidised, is between 0.5 and 1.8.

14. Process according to any one of Claims 1 to 13, characterised in that the base used is a hydroxide, an alcoholate or a salt of a weak acid with an alkali metal base.

15. Process according to any one of Claims 1 to 14, characterised in that the alkali metal base is sodium hydroxide or potassium hydroxide.

16. Process according to any one of Claims 1 to 15, characterised in that the amount of base is determined so that the pH of the reaction medium remains within the defined limits.

17. Process according to any one of Claims 1 to 16, characterised in that the pH of the reaction medium is between 2 and 6.

18. Process according to any one of Claims 1 to 17, characterised in that the hydroxybenzaldehyde subjected to oxidation with hydrogen peroxide has been obtained by a two-step process comprising the hydroxymethylation of a phenol to mono- and/or poly-methylolphenols by means of formaldehyde or a formaldehyde generator, followed by the oxidation of the mono- and/or poly-methylolphenols with molecular oxygen or a gas which contains molecular oxygen, in an alkaline aqueous phase, in the presence of a catalyst based on a noble metal and, if appropriate, on an activator taken from the group comprising Cd, Ce, Bi, Pb, Ag, Te and Sn, or their organic or inorganic derivatives.

19. Process according to Claim 18, characterised in that polyphenols of the formula :

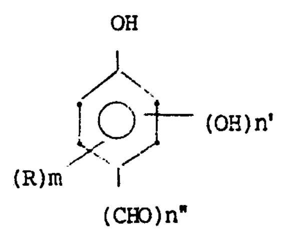

(II)

in which R, m, n' and n" have the meanings already indicated, are prepared by the hydroxymethylation of phenols of the formula :

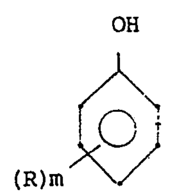

(VIII)

in wich R and m have the meanings already given, to hydroxymethylphenols of the formula :

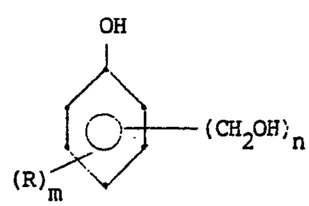

(IX)

in which R, m and n have the meanings already given, followed by the oxidation of the latter with molecular oxygen, in an alkaline aqueous phase, to hydroxybenzaldehydes of the formula :

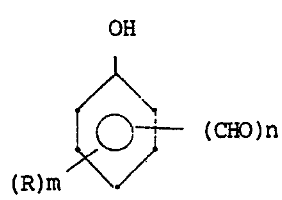

(I)

and finally the oxidation of the latter with hydrogen peroxide, in the aqueous phase, at pH ≤ 7.

20. Process according to Claim 18, characterised in that polyhydroxybenzaldehydes of the formula :

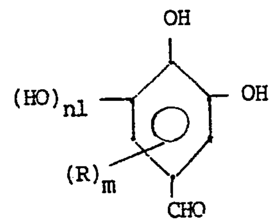

(VII)

in which R, m and $n_1$ have the meanings already given, are prepared by the hydroxymethylation of a phenol of the formula :

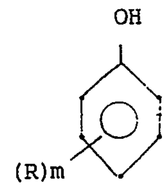

(VIII)

with formaldehyde or a formaldehyde generator, to polymethylolphenols of the formula :

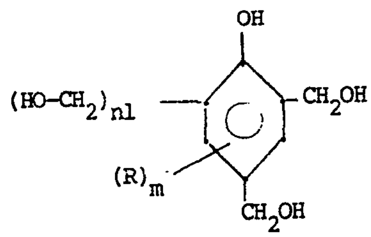

(X)

followed by oxidation of the latter with molecular oxygen, in an alkaline aqueous phase, to aldehydes of the general formula :

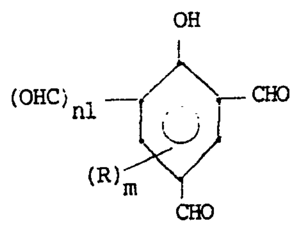

(VI)

and the oxidation of the latter with hydrogen peroxide, in the aqueous phase, at pH $\leq 7$, in the presence of an alkali metal base or alkaline earth metal base.

21. Process according to Claim 18, characterised in that 3,4,5-trihydroxybenzaldehyde is prepared from phenol by the hydroxymethylation of the latter to 2,4,6-trimethylolphenol, the oxidation of the trimethylolphenol with oxygen to 2,4,6-triformylphenol, and the oxidation of the latter with hydrogen peroxide, in the aqueous phase, at pH $\leq 7$, in the presence of an alkali metal base or alkaline earth metal base.

22. Process according to Claim 18, characterised in that 3,4-dihydroxy-5-methoxybenzaldehyde is prepared from guaiacol by the hydroxymethylation of the latter to 4,6-dihydroxymethyl-2-methoxyphenol, the oxidation of the dihydroxymethylguaiacol with molecular oxygen to diformylguaiacol, and the oxidation of the latter with hydrogen peroxide, in the aqueous phase, at pH $\leq 7$, in the presence of an alkali metal base or alkaline earth metal base.

23. Process according to any one of Claims 18 to 22, characterised in that the hydroxymethylation is carried out in the aqueous phase, in the presence of an alkali metal base or alkaline earth metal base, at a temperature of between 0 and 100 °C, the molar ratio of formaldehyde/phenol being between 0.1 and 4.

24. Process according to any one of Claims 18 to 23, characterised in that the oxidation of the mono- and/or poly-methylolphenols with molecular oxygen is carried out in an alkaline aqueous phase, at pH $\leq 8$, at a temperature of 10 to 100 °C, in the presence of 0.01 to 4 % by weight of platinum or palladium metal, relative to the methylolphenol, and, if appropriate, in the presence of 0.2 to 100 % by weight of an activator, relative to the noble metal.

25. Process according to any one of Claims 18 to 24, characterised in that the steps involving hydroxymethylation in the aqueous phase, oxidation of the mono- and/or poly-methylolphenols with molecular oxygen in an alkaline aqueous phase, and oxidation of the hydroxybenzaldehydes with hydrogen peroxide in the aqueous phase, at pH $\leq 7$, are carried out in succession without separation of the intermediates formed during the first two steps.

26. Process for the preparation of 3,4-dihydroxy-5-methoxybenzaldehyde, according to Claim 22, characterised in that :

a) in a first stage, dihydroxymethylguaiacol is prepared by reacting an aqueous solution of formaldehyde, or of a compound which generates formaldehyde, with an aqueous solution of an alkali metal guaiacolate, at a temperature of between 20 and 60 °C, the molar ratio of formaldehyde/guaiacol being between 2 and 4 and the molar ratio of alkali base/metal guaiacol being between 0.5 and 1.2,

b) in a second stage, a platinum-based catalyst deposited on an inert support and, if appropriate, containing an activator taken from the group comprising Cd, Ce, Bi, Pb, Ag, Te and Sn, or their derivatives, is added to the aqueous solution of alkali metal dihydroxymethylguaiacolate obtained in step a), the alkali metal dihydroxymethylguaiacolate is oxidised to the alkali metal diformylguaiacolate with molecular oxygen or a gas which contains molecular oxygen, at a temperature of between 30 and 60 °C, the pH of the medium being between 11.5 and 12, and the catalyst is then separated off, and

c) in a third stage, the alkali metal diformylguaiacolate is oxidised by the gradual addition of hydrogen peroxide to the aqueous solution obtained in stage b), after adjustment of the pH to a value of between 4 and 5 by adding a strong acid, the temperature being between 30 and 80 °C and the pH being kept between 4 and 5 by running in an alkali metal base, and the molar ratio of hydrogen peroxide/alkali metal diformylguaiacolate being between 1 and 1.5.

27. Process according to Claim 26, characterised in that stages a) and c) are carried out under an inert gas atmosphere.

28. Process according to any one of Claims 24 to 27, characterised in that, after removal of the catalyst at the end of stage b), the alkaline solution of alkali metal diformylguaiacolate is acidified to a pH which is less than or equal to 3.5, in order to precipitate the diformylguaiacol, the latter is separated from the aqueous phase and then suspended in water, the pH is brought to a value of between 4 and 5 and oxidation is carried out with hydrogen peroxide.

**Ansprüche** (für die Vertragsstaaten : BE, CH, DE, FR, GB, IT, LI, LU, NL, SE)

1. Verfahren zur Herstellung von Polyphenolen, (d. h. mehrwertigen Phenolen) die gegebenenfalls eine Aldehydgruppe enthalten, mittels Oxidation von Hydroxybenzaldehyden, die mindestens eine Aldehydgruppe in o- und/oder p-Stellung zur Hydroxylgruppe enthalten und der allgemeinen Formel

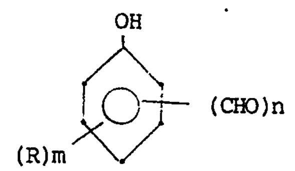

(I)

entsprechen, in der

— n eine ganze Zahl von 1 bis 3 ist,

— R für eine Alkyl-, Alkoxy-, Hydroxyalkyl-, Cycloalkyl-, Aryl-, Alkoxyalkyl- oder Nitrogruppe oder für ein Halogenatom steht,

— m eine ganze Zahl von 0 bis 3 ist, wobei die Summe aus m + n höchstens gleich 4 ist, ausgenommen die Monohydroxybenzaldehyde der Formel (I), in der n gleich 1 ist, mit Wasserstoffperoxid in wäßrigem Medium in Gegenwart einer Alkali- oder Erdalkalibase, dadurch gekennzeichnet, daß der pH-Wert des Reaktionsmediums während der Reaktionsdauer unter 7 oder bei 7 gehalten wird.

2. Verfahren zur Herstellung von Polyphenolen nach Anspruch 1, dadurch gekennzeichnet, daß die erhaltenen Phenole der Formel

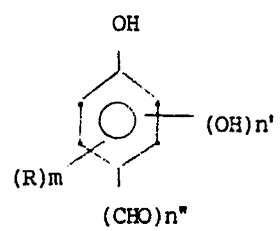

(II)

entsprechen, in der

— R und m die bereits angegebene Bedeutung haben,

— n' eine ganze Zahl von 1 bis 2 ist,

— n'' 0 oder 1 ist, wobei die Summe aus n' und n'' gleich n ist.

3. Verfahren nach den Ansprüchen 1 und 2, dadurch gekennzeichnet, daß in den Formeln (I) und (II) R für eine niedere Alkylgruppe, eine niedere Alkoxygruppe oder eine Hydroxylgruppe steht.

4. Verfahren nach einem der Ansprüche 1 bis 3, dadurch gekennzeichnet, daß n 2 oder 3 ist.

5. Verfahren nach einem der Ansprüche 1 bis 4, dadurch gekennzeichnet, daß m 0 oder 1 ist.

6. Verfahren nach Anspruch 1, dadurch gekennzeichnet, daß man Polyhydroxybenzaldehyde der allgemeinen Formel

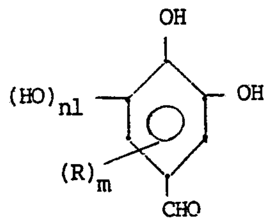

(VII)

herstellt, in der R, m die angegebene Bedeutung haben, und $n_1$ 0 oder 1 ist, mittels Oxidation eines Hydroxybenzaldehyds der allgemeinen Formel

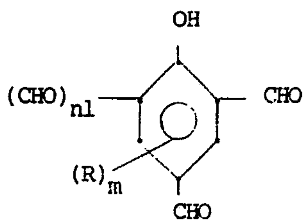

(VI)

7. Verfahren nach Anspruch 1, dadurch gekennzeichnet, daß man 1,2,4-Trihydroxybenzol durch Oxidation von Protocatechualdehyd herstellt.

8. Verfahren nach Anspruch 1, dadurch gekennzeichnet, daß man Pyrogallol durch Oxidation von 2,3-Dihydroxybenzaldehyd herstellt.

9. Verfahren nach Anspruch 1, dadurch gekennzeichnet, daß man Pyrogallol und 1,2,4-Trihydroxybenzol durch Oxidation eines Gemisches aus Protocatechualdehyd und 2,3-Dihydroxybenzaldehyd herstellt.

10. Verfahren nach Anspruch 1, dadurch gekennzeichnet, daß man 3,4-Dihydroxy-5-methoxybenzaldehyd durch Oxidation von 4,6-Diformylguajacol herstellt.

11. Verfahren nach Anspruch 1, dadurch gekennzeichnet, daß man 3,4,5-Trihydroxybenzaldehyd durch Oxidation von 2,4,6-Triformylphenol herstellt.

12. Verfahren nach einem der Ansprüche 1 bis 11, dadurch gekennzeichnet, daß die Reaktionstemperatur zwischen 0 und 100 °C liegt.

13. Verfahren nach einem der Ansprüche 1 bis 12, dadurch gekennzeichnet, daß die Menge Wasserstoffperoxid, angegeben in Mole $H_2O_2$ je Aldehydgruppe, die oxidiert werden soll, 0,5 bis 1,8 beträgt.

14. Verfahren nach einem der Ansprüche 1 bis 13, dadurch gekennzeichnet, daß die verwendete Base ein Hydroxid, ein Alkoholat oder ein Salz einer schwachen Säure mit einer Alkalibase ist.

15. Verfahren nach einem der Ansprüche 1 bis 14, dadurch gekennzeichnet, daß die Alkalibase Natriumhydroxid oder Kaliumhydroxid ist.

16. Verfahren nach einem der Ansprüche 1 bis 15, dadurch gekennzeichnet, daß die Menge der Base so bestimmt wird, daß der pH-Wert des Reaktionsmediums innerhalb der angegebenen Grenzen bleibt.

17. Verfahren nach einem der Ansprüche 1 bis 16, dadurch gekennzeichnet, daß der pH-Wert des Reaktionsmediums 2 bis 6 beträgt.

18. Verfahren nach einem der Ansprüche 1 bis 17, dadurch gekennzeichnet, daß der Hydroxybenzaldehyd, der der Oxidation mit Wasserstoffperoxid unterworfen wird, erhalten worden ist durch ein 2-stufiges Verfahren, umfassend die Hydroxymethylierung eines Phenols zu Mono- und/oder Polymethylolphenolen mit Hilfe von Formaldehyd oder einer Formaldehyd erzeugenden Verbindung und anschließende Oxidation in wäßrig-alkalischer Phase der Mono- und/oder Polymethylolphenole durch molekularen Sauerstoff oder ein Gas, das diesen enthält, in Gegenwart eines Katalysators auf Edelmetallbasis und gegebenenfalls eines Aktivators aus der Gruppe Cd, Ce, Bi, Pb, Ag, Te und Sn oder deren organischen oder anorganischen Verbindungen.

19. Verfahren nach Anspruch 18, dadurch gekennzeichnet, daß man Polyphenole der Formel

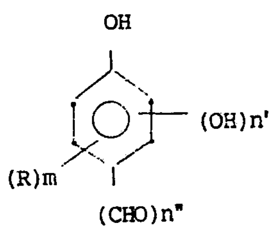

(II)

herstellt, in der R, m, n' und n" die bereits angegebene Bedeutung haben, durch Hydroxymethylierung von Phenolen der Formel

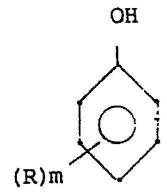

(VIII)

in der R und n die bereits angegebene Bedeutung haben, zu Hydroxymethylphenolen der Formel

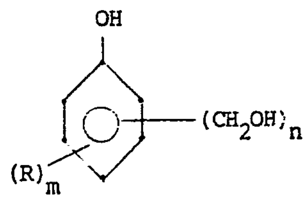

(IX)

in der R, m und n die bereits angegebene Bedeutung haben, und Oxidation dieser letzteren mit molekularem Sauerstoff in wäßrig-alkalischer Phase zu Hydroxybenzaldehyden der Formel

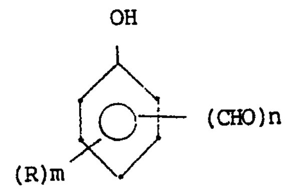

(I)

und schließlich Oxidation dieser letzteren mit Wasserstoffperoxid in wäßriger Phase bei pH $\leq 7$.

20. Verfahren nach Anspruch 18, dadurch gekennzeichnet, daß man Polyhydroxybenzaldehyde der Formel

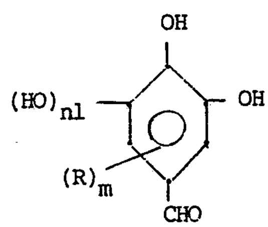

(VII)

in der R, m und $n_1$ die bereits angegebene Bedeutung haben, herstellt durch Hydroxymethylierung eines Phenols der Formel

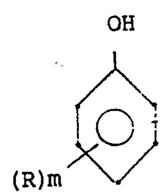

(VIII)

mit Formaldehyd oder einer Formaldehyd erzeugenden Verbindung zu Polymethylolphenolen der Formel

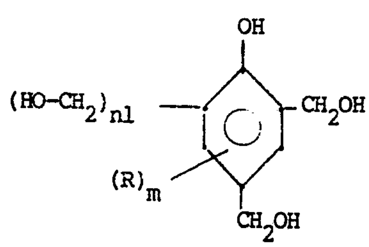

(X)

und Oxidation dieser letzteren mit molekularem Sauerstoff in wäßrig-alkalischer Phase zu Aldehyden der allgemeinen Formel

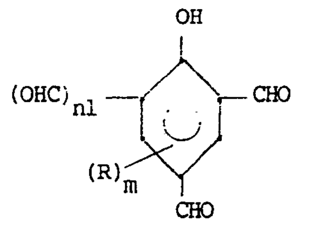

(VI)

sowie Oxidation dieser letzteren mit Wasserstoffperoxid in wäßriger Phase bei pH ≤ 7 in Gegenwart einer Alkali- oder Erdalkalibase.

21. Verfahren nach Anspruch 18, dadurch gekennzeichnet, daß man 3,4,5-Trihydroxybenzaldehyd, ausgehend von Phenol herstellt, durch Hydroxymethylierung des letzteren zu 2,4,6-Trimethylolphenol, Oxidation des Trimethylolphenols mit Sauerstoff zu 2,4,6-Triformylphenol und Oxidation dieser letzteren Verbindung mit Wasserstoffperoxid in wäßriger Phase bei pH ≤ 7 in Gegenwart einer Alkali- oder Erdalkalibase.

22. Verfahren nach Anspruch 18, dadurch gekennzeichnet, daß man 3,4-Dihydroxy-5-methoxybenzaldehyd, ausgehend von Guajacol herstellt durch Hydroxymethylierung des letzteren zu 4,6-Dihydroxymethyl-2-methoxyphenol, Oxidation des Dihydroxymethylguajacols durch molekularen Sauerstoff zu Diformylguajakol und Oxidation dieser letzteren Verbindung mit Wasserstoffperoxid in wäßriger Phase bei pH ≤ 7 in Gegenwart einer Alkali- oder Erdalkalibase.

23. Verfahren nach einem der Ansprüche 18 bis 22, dadurch gekennzeichnet, daß die Hydroxymethylierung in wäßriger Phase in Gegenwart einer Alkali- oder Erdalkalibase bei einer Temperatur von 0 bis 100 °C durchgeführt wird, wobei das Molverhältnis von Formaldehyd zu Phenol 0,1 bis 4 beträgt.

24. Verfahren nach einem der Ansprüche 18 bis 23, dadurch gekennzeichnet, daß die Oxidation der Mono- und/oder Polymethylolphenole mit molekularem Sauerstoff in wäßrig-alkalischer Phase bei pH ≥ 8, bei einer Temperatur von 10 bis 100 °C in Gegenwart von 0,01 bis 4 Gew.-% metallischem Platin oder Palladium, bezogen auf das Methylolphenol, sowie gegebenenfalls 0,2 bis 100 Gew.-%, bezogen auf das Edelmetall, eines Aktivators durchgeführt wird.

25. Verfahren nach einem der Ansprüche 18 bis 24, dadurch gekennzeichnet, daß die Stufen der Hydroxymethylierung in wäßriger Phase, der Oxidation der Mono- und/oder Polymethylolphenole mit molekularem Sauerstoff in wäßrig-alkalischer Phase und der Oxidation mit Wasserstoffperoxid der Hydroxybenzaldehyde in wäßriger Phase bei pH ≤ 7 aufeinanderfolgend ohne Isolierung der in den beiden ersten Stufen entstandenen Zwischenverbindungen durchgeführt werden.

26. Verfahren zur Herstellung von 3,4-Dihydroxy-5-methoxybenzaldehyd nach Anspruch 22, dadurch gekennzeichnet, daß man

a) in einer ersten Stufe Dihydroxymethylguajacol durch Reaktion einer wäßrigen Lösung von Formaldehyd oder einer Formaldehyd erzeugenden Verbindung mit einer wäßrigen Lösung eines Alkaliguajacolats bei einer Temperatur von 20 bis 60 °C herstellt, wobei das Molverhältnis von Formaldehyd zu Guajacol 2 bis 4 und das Molverhältnis von Alkalibase zu Guajacol 0,5 bis 1,2 beträgt,

b) in einer zweiten Stufe zu der in der Stufe (a) erhaltenen wäßrigen Lösung von Dihydroxymethylguajacolat einen Katalysator auf der Basis von Platin, abgeschieden auf einen inerten Träger, und umfassend gegebenenfalls einen Aktivator aus der Gruppe Cd, Ce, Bi, Pb, Ag, Te und Sn oder deren Verbindungen zugibt und die Oxidation des Alkali-dihydroxymethylguajacolats zu Alkali-diformylguajacolat mit molekularem Sauerstoff oder einem Gas, das diesen enthält, bei einer Temperatur von 30 bis 60 °C und bei einem pH-Wert des Mediums von 11,5 bis 12 vornimmt und den Katalysator abtrennt,

c) in einer dritten Stufe das Alkali-diformylguajacolat oxidiert durch allmähliche Zugabe von Wasserstoffperoxid zu der in der Stufe (b) erhaltenen wäßrigen Lösung, nachdem der pH-Wert durch Zugabe einer starken Säure auf einen Wert von 4 bis 5 eingestellt worden ist, wobei die Temperatur 30 bis 80 °C beträgt und der pH-Wert durch Zulaufenlassen einer Alkalibase zwischen 4 und 5 gehalten wird und wobei das Molverhältnis von Wasserstoffperoxid zu Alkalidiformylguajacolat 1 bis 1,5 beträgt.

27. Verfahren nach Anspruch 26, dadurch gekennzeichnet, daß die Stufen (a) und (c) in einer Inertgasatmosphäre durchgeführt werden.

28. Verfahren nach einem der Ansprüche 24 bis 27, dadurch gekennzeichnet, daß man nach Abtrennen des Katalysators am Ende der Stufe (b) die alkalische Lösung des Alkali-diformylguajacolats auf einen pH-Wert von 3,5 oder darunter ansäuert, um Diformylguajacol auszufällen, dieses letztere von der wäßrigen Phase abtrennt und in Wasser supendiert, den pH-Wert auf einen Wert von 4 bis 5 bringt und die Oxidation mit Wasserstoffperoxid durchführt.

29. Neues Polyformylphenol, dadurch gekennzeichnet, daß es das 2,4,6-Triformylphenol ist.

**Ansprüche** (für den Vertragsstaat AT)

1. Verfahren zur Herstellung von Polyphenolen, (d. h. mehrwertigen Phenolen) die gegebenenfalls eine Aldehydgruppe enthalten, mittels Oxidation von Hydroxybenzaldehyden, die mindestens eine Aldehydgruppe in o- und/oder p-Stellung zur Hydroxylgruppe enthalten und der allgemeinen Formel

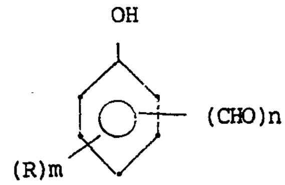

(I)

entsprechen, in der

— n eine ganze Zahl von 1 bis 3 ist,

— R für eine Alkyl-, Alkoxy-, Hydroxyalkyl-, Cycloalkyl-, Aryl-, Alkoxyalkyl- oder Nitrogruppe oder für ein Halogenatom steht,

— m eine ganze Zahl von 0 bis 3 ist, wobei die Summe aus m + n höchstens gleich 4 ist, ausgenommen die Monohydroxybenzaldehyde der Formel (I), in der n gleich 1 ist, mit Wasserstoffperoxid in wäßrigem Medium in Gegenwart einer Alkali- oder Erdalkalibase, dadurch gekennzeichnet, daß der pH-Wert des Reaktionsmediums während der Reaktionsdauer unter 7 oder bei 7 gehalten wird.

2. Verfahren zur Herstellung von Polyphenolen nach Anspruch 1, dadurch gekennzeichnet, daß die erhaltenen Phenole der Formel

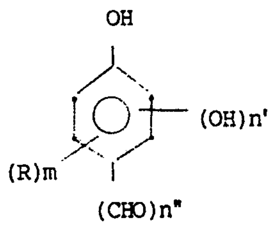

(II)

entsprechen, in der

— R und m die bereits angegebene Bedeutung haben,

— n' eine ganze Zahl von 1 bis 2 ist,

— n" 0 oder 1 ist, wobei die Summe aus n' und n" gleich n ist.

3. Verfahren nach den Ansprüchen 1 und 2, dadurch gekennzeichnet, daß in den Formeln (I) und (II) R für eine niedere Alkylgruppe, eine niedere Alkoxygruppe oder eine Hydroxylgruppe steht.

4. Verfahren nach einem der Ansprüche 1 bis 3, dadurch gekennzeichnet, daß n 2 oder 3 ist.

5. Verfahren nach einem der Ansprüche 1 bis 4, dadurch gekennzeichnet, daß m 0 oder 1 ist.

6. Verfahren nach Anspruch 1, dadurch gekennzeichnet, daß man Polyhydroxybenzaldehyde der allgemeinen Formel

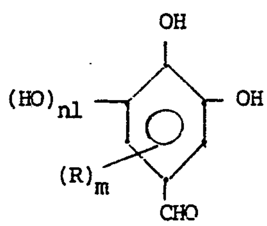

(VII)

herstellt, in der R, m die angegebene Bedeutung haben, und $n_1$ 0 oder 1 ist, mittels Oxidation eines Hydroxybenzaldehyds der allgemeinen Formel

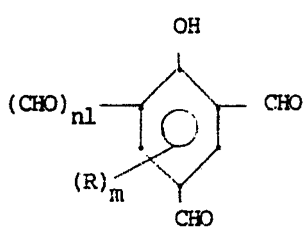

(VI)

7. Verfahren nach Anspruch 1, dadurch gekennzeichnet, daß man 1,2,4-Trihydroxybenzol durch Oxidation von Protocatechualdehyd herstellt.

8. Verfahren nach Anspruch 1, dadurch gekennzeichnet, daß man Pyrogallol durch Oxidation von 2,3-Dihydroxybenzaldehyd herstellt.

9. Verfahren nach Anspruch 1, dadurch gekennzeichnet, daß man Pyrogallol und 1,2,4-Trihydroxybenzol durch Oxidation eines Gemisches aus Protocatechualdehyd und 2,3-Dihydroxybenzaldehyd herstellt.

10. Verfahren nach Anspruch 1, dadurch gekennzeichnet, daß man 3,4-Dihydroxy-5-methoxybenzaldehyd durch Oxidation von 4,6-Diformylguajacol herstellt.

11. Verfahren nach Anspruch 1, dadurch gekennzeichnet, daß man 3,4,5-Trihydroxybenzaldehyd durch Oxidation von 2,4,6-Triformylphenol herstellt.

12. Verfahren nach einem der Ansprüche 1 bis 11, dadurch gekennzeichnet, daß die Reaktionstemperatur zwischen 0 und 100 °C liegt.

13. Verfahren nach einem der Ansprüche 1 bis 12, dadurch gekennzeichnet, daß die Menge Wasserstoffperoxid, angegeben in Mole $H_2O_2$ je Aldehydgruppe, die oxidiert werden soll, 0,5 bis 1,8 beträgt.

14. Verfahren nach einem der Ansprüche 1 bis 13, dadurch gekennzeichnet, daß die verwendete Base ein Hydroxid, ein Alkoholat oder ein Salz einer schwachen Säure mit einer Alkalibase ist.

15. Verfahren nach einem der Ansprüche 1 bis 14, dadurch gekennzeichnet, daß die Alkalibase Natriumhydroxid oder Kaliumhydroxid ist.

16. Verfahren nach einem der Ansprüche 1 bis 15, dadurch gekennzeichnet, daß die Menge der Base so bestimmt wird, daß der pH-Wert des Reaktionsmediums innerhalb der angegebenen Grenzen bleibt.

17. Verfahren nach einem der Ansprüche 1 bis 16, dadurch gekennzeichnet, daß der pH-Wert des Reaktionsmediums 2 bis 6 beträgt.

18. Verfahren nach einem der Ansprüche 1 bis 17, dadurch gekennzeichnet, daß der Hydroxybenzaldehyd, der der Oxidation mit Wasserstoffperoxid unterworfen wird, erhalten worden ist durch ein 2-stufiges Verfahren, umfassend die Hydroxymethylierung eines Phenols zu Mono- und/oder Polymethylolphenolen mit Hilfe von Formaldehyd oder einer Formaldehyd erzeugenden Verbindung und anschließende Oxidation in wäßrig-alkalischer Phase der Mono- und/oder Polymethylolphenole durch molekularen Sauerstoff oder ein Gas, das diesen enthält, in Gegenwart eines Katalysators auf Edelmetallbasis und gegebenenfalls eines Aktivators aus der Gruppe Cd, Ce, Bi, Pb, Ag, Te und Sn oder deren organischen oder anorganischen Verbindungen.

19. Verfahren nach Anspruch 18, dadurch gekennzeichnet, daß man Polyphenole der Formel

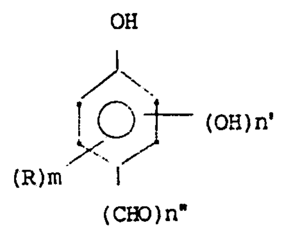

(II)

herstellt, in der R, m, n' und n'' die bereits angegebene Bedeutung haben, durch Hydroxymethylierung von Phenolen der Formel

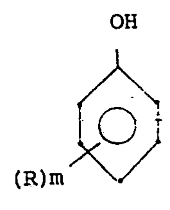

(VIII)

in der R und n die bereits angegebene Bedeutung haben, zu Hydroxymethylphenolen der Formel

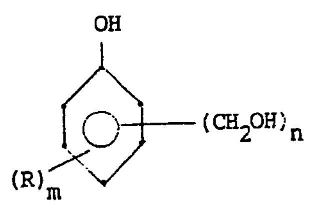

(IX)

in der R, m und n die bereits angegebene Bedeutung haben, und Oxidation dieser letzteren mit molekularem Sauerstoff in wäßrig-alkalischer Phase zu Hydroxybenzaldehyden der Formel

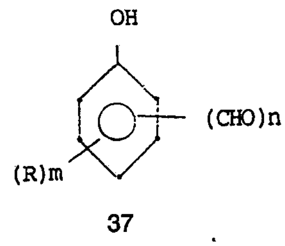

(I)

und schließlich Oxidation dieser letzteren mit Wasserstoffperoxid in wäßriger Phase bei pH ≤ 7.

20. Verfahren nach Anspruch 18, dadurch gekennzeichnet, daß man Polyhydroxybenzaldehyde der Formel

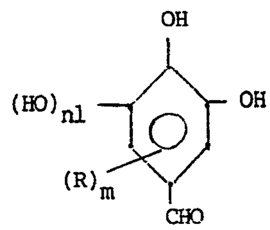

(VII)

in der R, m und $n_1$ die bereits angegebene Bedeutung haben, herstellt durch Hydroxymethylierung eines Phenols der Formel

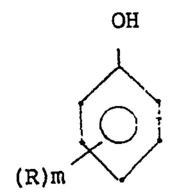

(VIII)

mit Formaldehyd oder einer Formaldehyd erzeugenden Verbindung zu Polymethylolphenolen der Formel

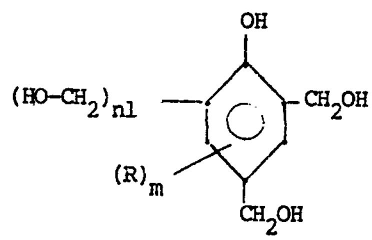

(X)

und Oxidation dieser letzteren mit molekularem Sauerstoff in wäßrig-alkalischer Phase zu Aldehyden der allgemeinen Formel

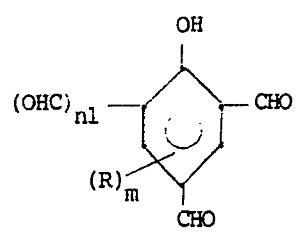

(VI)

sowie Oxidation dieser letzteren mit Wasserstoffperoxid in wäßriger Phase bei pH ≤ 7 in Gegenwart einer Alkali- oder Erdalkalibase.

21. Verfahren nach Anspruch 18, dadurch gekennzeichnet, daß man 3,4,5-Trihydroxybenzaldehyd, ausgehend von Phenol herstellt, durch Hydroxymethylierung des letzteren zu 2,4,6-Trimethylolphenol, Oxidation des Trimethylolphenols mit Sauerstoff zu 2,4,6-Triformylphenol und Oxidation dieser letzteren Verbindung mit Wasserstoffperoxid in wäßriger Phase bei pH ≤ 7 in Gegenwart einer Alkali- oder Erdalkalibase.

22. Verfahren nach Anspruch 18, dadurch gekennzeichnet, daß man 3,4-Dihydroxy-5-methoxybenzaldehyd, ausgehend von Guajacol herstellt durch Hydroxymethylierung des letzteren zu 4,6-Dihydroxymethyl-2-methoxyphenol, Oxidation des Dihydroxymethylguajacols durch molekularen Sauerstoff zu Diformylguajakol und Oxidation dieser letzteren Verbindung mit Wasserstoffperoxid in wäßriger Phase bei pH ≤ 7 in Gegenwart einer Alkali- oder Erdalkalibase.

23. Verfahren nach einem der Ansprüche 18 bis 22, dadurch gekennzeichnet, daß die Hydroxymethylierung in wäßriger Phase in Gegenwart einer Alkali- oder Erdalkalibase bei einer Temperatur von 0 bis 100 °C durchgeführt wird, wobei das Molverhältnis von Formaldehyd zu Phenol 0,1 bis 4 beträgt.

24. Verfahren nach einem der Ansprüche 18 bis 23, dadurch gekennzeichnet, daß die Oxidation der Mono- und/oder Polymethylolphenole mit molekularem Sauerstoff in wäßrig-alkalischer Phase bei pH ≥ 8, bei einer Temperatur von 10 bis 100 °C in Gegenwart von 0,01 bis 4 Gew.-% metallischem Platin oder

Palladium, bezogen auf das Methylolphenol, sowie gegebenenfalls 0,2 bis 100 Gew.-%, bezogen auf das Edelmetall, eines Aktivators durchgeführt wird.

25. Verfahren nach einem der Ansprüche 18 bis 24, dadurch gekennzeichnet, daß die Stufen der Hydroxymethylierung in wäßriger Phase, der Oxidation der Mono- und/oder Polymethylolphenole mit molekularem Sauerstoff in wäßrig-alkalischer Phase und der Oxidation mit Wasserstoffperoxid der Hydroxybenzaldehyde in wäßriger Phase bei pH $\leq 7$ aufeinanderfolgend ohne Isolierung der in den beiden ersten Stufen entstandenen Zwischenverbindungen durchgeführt werden.

26. Verfahren zur Herstellung von 3,4-Dihydroxy-5-methoxybenzaldehyd nach Anspruch 22, dadurch gekennzeichnet, daß man

a) in einer ersten Stufe Dihydroxymethylguajacol durch Reaktion einer wäßrigen Lösung von Formaldehyd oder einer Formaldehyd erzeugenden Verbindung mit einer wäßrigen Lösung eines Alkaliguajacolats bei einer Temperatur von 20 bis 60 °C herstellt, wobei das Molverhältnis von Formaldehyd zu Guajacol 2 bis 4 und das Molverhältnis von Alkalibase zu Guajacol 0,5 bis 1,2 beträgt,

b) in einer zweiten Stufe zu der in der Stufe (a) erhaltenen wäßrigen Lösung von Dihydroxymethylguajacolat einen Katalysator auf der Basis von Platin, abgeschieden auf einem inerten Träger, und umfassend gegebenenfalls einen Aktivator aus der Gruppe Cd, Ce, Bi, Pb, Ag, Te und Sn oder deren Verbindungen zugibt und die Oxidation des Alkali-dihydroxymethylguajacolats zu Alkali-diformylguajacolat mit molekularem Sauerstoff oder einem Gas, das diesen enthält, bei einer Temperatur von 30 bis 60 °C und bei einem pH-Wert des Mediums von 11,5 bis 12 vornimmt und den Katalysator abtrennt,

c) in einer dritten Stufe das Alkali-diformylguajacolat oxidiert durch allmähliche Zugabe von Wasserstoffperoxid zu der in der Stufe (b) erhaltenen wäßrigen Lösung, nachdem der pH-Wert durch Zugabe einer starken Säure auf einen Wert von 4 bis 5 eingestellt worden ist, wobei die Temperatur 30 bis 80 °C beträgt und der pH-Wert durch Zulaufenlassen einer Alkalibase zwischen 4 und 5 gehalten wird und wobei das Molverhältnis von Wasserstoffperoxid zu Alkalidiformylguajacolat 1 bis 1,5 beträgt.

27. Verfahren nach Anspruch 26, dadurch gekennzeichnet, daß die Stufen (a) und (c) in einer Inertgasatmosphäre durchgeführt werden.

28. Verfahren nach einem der Ansprüche 24 bis 27, dadurch gekennzeichnet, daß man nach Abtrennen des Katalysators am Ende der Stufe (b) die alkalische Lösung des Alkali-diformylguajacolats auf einen pH-Wert von 3,5 oder darunter ansäuert, um Diformylguajacol auszufällen, dieses letztere von der wäßrigen Phase abtrennt und in Wasser supendiert, den pH-Wert auf einen Wert von 4 bis 5 bringt und die Oxidation mit Wasserstoffperoxid durchführt.